# EUROPEAN PATENT SPECIFICATION

(11) **EP 2 596 136 B1**
(45) Date of publication and mention of the grant of the patent: **16.07.2014**
(21) Application number: 11865138.9
(22) Date of filing: 06.05.2011
(51) Int. Cl.: C12Q 1/68, G01N 33/48, C12N 15/113

(54) **MARKER CONSISTING OF PLASMA MICRORNA AND NEW METHOD FOR DIAGNOSIS OF HEPATOCELLULAR CARCINOMA**
MARKER AUS PLASMA-MIKRO-RNA UND NEUES VERFAHREN ZUR DIAGNOSTIK DES HEPATOZELLULÄREN KARZINOMS
MARQUEUR CONSTITUÉ D'UN MICROARN DU PLASMA ET NOUVELLE MÉTHODE DE DIAGNOSTIC D'UN CARCINOME HÉPATOCELLULAIRE

(43) Date of publication of application: 29.05.2013
(73) Proprietor: Zhongshan Hospital Fudan University, Shanghai 200032 (CN)
(72) Inventor: FAN, Jia, Shanghai 200032 (CN); ZHOU, Jian, Shanghai 200032 (CN); DAI, Zhi, Shanghai 200032 (CN); YU, Lei, Shanghai 200032 (CN); HU, Jie, Shanghai 200032 (CN); WANG, Zheng, Shanghai 200032 (CN)
(74) Representative: Nevant, Marc
(86) International application number: PCT/CN2011/073774
(87) International publication number: WO 2012/151736

(56) References cited:
- WO-A1-2009/036236
- WO-A1-2010/043114
- WO-A2-2010/055488
- WO-A2-2010/055488
- CN-A- 101 368 213
- CN-A- 102 021 169
- KUTAY H. ET AL.: 'Downregulation of miR-122 in the rodent and human hepatocellular carcinomas' JOURNAL OF CELLULAR BIOCHEMISTRY vol. 99, no. IS.3, 2006, pages 671 - 678
- URA SHUNSUKE ET AL.: 'Differential microRNA expression between hepatitis B and hepatitis C leading disease progression to hepatocellular carcinoma' HEPATOLOGY vol. 49, no. 4, April 2009, pages 1098 - 1112
- JI JUNFANG ET AL.: 'MicroRNA Expression, Survival, and Response to Interferon in Liver Cancer' THE NEW ENGLAND JOURNAL OF MEDICINE. vol. 361, 08 October 2009, pages 1437 - 1447
- JIANG JINMAI ET AL.: 'Association of MicroRNA expression in hepatocellular carcinomas with hepatitis infection, cirrhosis, and patient survival' CLINICAL CANCER RESEARCH. vol. 14, no. 2, 25 January 2008, pages 419 - 427

## Description

### FIELD OF THE INVENTION

The present invention relates to a marker consisting of plasma microRNA (including hsa-miR-122, hsa-miR-192, hsa-miR-21, hsa-miR-223, hsa-miR-26a, hsa-miR-27a and hsa-miR-801) and a new method for diagnosis of hepatocellular carcinoma, particularly for early hepatocellular carcinoma (BCLC stage 0 and A).

### BACKGROUND OF THE INVENTION

Hepatocellular carcinoma (HCC) is one of the most common and rapidly fatal human malignancies worldwide. It represents the major histological type of liver cancer and likely accounts for 70%-85% of all cases of liver cancer. Approx. 500,000 new cases occur worldwide every year, with almost the same number of fatalities, reflecting the lack of effective early detection and treatment options (Thorgeirsson, S.S. and Grisham, J.W. (2002) Nat Genet 31, 339-346; Parkin, D.M. et al. (2005) CA Cancer J Clin 55, 74-108; Bosch F.X. et al (2004) Gastroenterology 127, S5-S16; Perz J.F. et al. (2006) J Hepatol 45, 529-538).

Hepatocellular carcinoma thus represents a type of an extremely poor prognostic cancer. The prognosis of patients depends on the stage when the disease is diagno sed. The 5-year survival in HCC patients without operation is < 5%, while the postoperative 5-year survival is 60%-70%. When tumor size is < 2 cm with surgical removal, the 5-year survival can be reached to 86%. However, the 3-year survival in early cancer patients (tumor size < 5 cm) without any treatment is only 17-21%. This illustrates the early cancer detection is critical for the treatment and the patient survival (Tang, Z.Y. (2001) World J Gastroenterol 7, 445-454; Chambers, A.F. et al. (2002) Nat Rev Cancer 2, 563-572; Motola-Kuba D. et al. (2006) Annals of Hepatology 5, 16-24).

Although early detection and surgical removal of hepatocellular tumors have significantly improved the patient survival in recent years, the majority of tumors are still not detected early in tumor progression, that is, in a non-fatal stage. Only about 10%-20% of patients with HCC, defined by parameters of relatively normal liver function and a manageable tumor lesion as determined by the available clinical staging systems, are currently eligible for surgical intervention. Moreover, patients who were resected often have a high frequency of metastasis/recurrence, and postoperative 5-year survival is only 30%-40%.

A definitive diagnosis of liver cancer is always based on histological confirmation. Tissue can be sampled with a needle aspiration or biopsy. However, some liver cancers are well differentiated, which means they are made up of nearly fully developed, mature hepatocytes. Therefore, these cancers can look very similar to non-cancerous liver tissue under a microscope. Moreover, not all pathologists are trained to recognize the subtle differences between well-differentiated liver cancer and normal liver tissue. Also, some pathologists can mistake liver cancer for adenocarcinoma in the liver. An adenocarcinoma is a different type of cancer, and it originates from outside of the liver. Most importantly, a metastatic adenocarcinoma would be treated differently from a primary liver cancer. Therefore, early detection of such tumors would be desirable in order to discriminate these different types of tumor and to guide the therapy decision in patients exhibiting HCC and thus can markedly help to improve long-term survival.

The most common risk of the aspiration or biopsy in liver tissue is bleeding, especially because liver cancer is a tumor that is very vascular (contains many blood vessels). In many instances, there is probably no need for a tissue diagnosis by biopsy or aspiration. If a patient has a risk factor for liver cancer (for example, cirrhosis, chronic hepatitis B, or chronic hepatitis C), a significantly elevated alpha-fetoprotein (AFP) blood level, meet defined imaging criteria, the doctor can be almost certain that the patient has liver cancer without doing a biopsy. Currently, AFP is only serum marker used for the early HCC detection (Mizejewski, G.J. (2003) Expert Rev Anticancer Ther 2, 709-735; Paul, S.B. et al. (2007) Oncology 72, Suppl. 1, 117-123). However, this single marker has a low sensitivity and is frequently inadequate because of false-positive results. The serum AFP test can readily detect hepatocellular tumors in only 60% of the patients. On the other hand, in a large number of cirrhosis patients, AFP can be elevated in the absence of cancerous states. Therefore, there is a significant unmet need for the identification of novel molecular markers and development of sensitive blood-based tests for early detection and differential diagnosis of hepatocellular carcinoma.

Several classification systems are available for hepatocellular carcinoma. The Barcelona Clinic Liver Cancer (BCLC) classification (Llovet, J.M. (2003) Lancet 362, 1907-1917) has emerged during recent years as the standard classification for clinical management of patients with hepatocellular cancer. The BCLC classification links stage stratification with a recommended treatment strategy and defines standard of care for each tumor stage (Llovet, J.M. (2008) J Natl Cancer Inst 100, 698-711). Patients with very early HCC (stage 0) are optimal candidates for resection. Patients with early HCC (stage A) are candidates for radical therapy (resection, liver transplantation or local ablation). Patients with intermediate HCC (stage B) benefit from transarterial chemoembolization (TACE). Patients with advanced HCC (stage C) benefit from sorafenib therpay. Patients with end-stage disease (stage D) will receive symptomatic treatment.

Many diagnostic assays are hampered by the fact that they are typically based on the analysis of only a single molecular marker, which might affect detection reliability and/or accuracy. In addition, a single marker normally does not enable detailed predictions concerning latency stages, tumor progression, and the like. Thus, there is still a continuing need for the identification of alternative molecular markers and assay formats overcoming these limitations.

One approach to address this issue might be based on small regulatory RNA molecules, in particular on microRNAs (miRNAs) which, constitute an evolutionary conserved class of endogenously expressed small non-coding RNAs of 20-25 nucleotides (nt) in size that can mediate the expression of target mRNAs and thus - since their discovery about ten years ago - have been implicated with critical functions in cellular development, differentiation, proliferation, and apoptosis (Bartel, D.P. (2004) Cell 116, 281-297, Ambros, V. (2004) Nature 431, 350-355; He, L. et al. (2004) Nat Rev Genet 5, 522-531). Furthermore, miRNAs have advantages over mRNAs as cancer biomarkers, since they are very stable in vitro and long-lived in vivo (Lu, J. et al., (2005) Nature 435, 834-838; Lim, L.P. et al., (2005) Nature 433, 769-773).

MicroRNAs are produced from primary transcripts that are processed to stem-loop structured precursors (pre-miRNAs) by the RNase III Drosha. After transport to the cytoplasm, another RNase III termed Dicer cleaves of the loop of the pre-miRNA hairpin to form a short double-stranded (ds) RNA, one strand of which is incorporated as mature miRNA into a miRNA-protein (miRNP). The miRNA guides the miRNPs to their target mRNAs where they exert their function (Bartel, D.P. (2004) Cell 23, 281-292; He, L. and Hannon, G.J. (2004) Nat Rev Genet 5, 522-531).

Depending on the degree of complementarity between the miRNA and its target, miRNAs can guide different regulatory processes. Target mRNAs that are highly complementary to miRNAs are specifically cleaved by mechanisms identical to RNA interference (RNAi). Thus, in such scenario, the miRNAs function as short interfering RNAs (siRNAs). Target mRNAs with less complementarity to miRNAs are either directed to cellular degradation pathways or are translationally repressed without affecting the mRNA level. However, the mechanism of how microRNAs repress translation of their target mRNAs is still a matter of controversy.

High-throughput microRNA quantification technologies, such as microRNA microarray, real-time RT-PCR-based TaqMan microRNA assays, have provided powerful tools to study the global microRNA profile in whole cancer genome. Emerging data available indicate that dysregulation of microRNA expression may inter alia be associated with the development and/or progression of certain types of cancer. For example, two microRNAs , hsa-miR-15 and hsa-miR-16-1, were shown to map to a genetic locus that is deleted in chronic lymphatic leukemia (CLL) and it was found that in about 70% of the CLL patients, both microRNA genes are deleted or down-regulated. Furthermore, down-regulation of hsa-miR-143 and hsa-miR-145 was observed in colorectal neoplasia, whereas expression of the miRNAs let-7 is frequently reduced in lung cancers (Michael, M.Z. et al. (2003) Mol Cancer Res 1, 882-891; Mayr, C. et al. (2007) Science 315, 1576-1579). In fact, it has been speculated based on cancer-associated alterations in microRNA expression and the observation that microRNAs are frequently located at genomic regions involved in cancers that microRNAs may act both as tumor suppressors and as oncogenes (Esquela-Kerscher, A. and Slack, F.J (2006) Nat Rev Cancer 6, 259-269; Calin, G.A. and Croce, C.M. (2007) J Clin Invest 117, 2059-2066; Blenkiron, C. and Miska, E.A. (2007) Hum Mol Genet 16, R106-R113). Demonstrated abnormal expression patterns of microRNAs in human cancers highlight their potential use as diagnostic and prognostic biomarkers.

Several studies have reported microRNA expression profiling in human hepatocellular carcinoma (Murakami, Y. et al. (2006) Oncogene 25, 2537-2545; Li, W. et al. (2008) Int J Cancer 123, 1616-1622; Huang, Y.S. et al. (2008) Hepatology 23, 87-94; Ladeiro, Y. et al. (2008) Hepatology 47, 1955-1963; Jiang, J. et al. (2008) Clin Cancer Res 14, 419-427). Consistently, these studies have shown that specific microRNAs are aberrantly expressed in malignant cells or tissues as compared to nonmalignant hepatocytes or tissue. Thus, such microRNAs may provide insights into cellular processes involved in malignant transformation and progression.

Among the many possible types of samples, blood is thought to be ideal for screening high risk individuals, leading to early detection, diagnosis and monitoring the efficient treatment of cancers- since blood can be collected easily in a minimally invasive manner. It has been demonstrated that tumor-derived microRNAs are present in human plasma or serum in a remarkably stable form that is protected from endogenous RNase activity. These tumor-derived microRNAs in serum or plasma are at levels sufficient to be measurable as biomarkers for cancer detection. Moreover, the levels of plasma and serum microRNAs correlate strongly, suggesting that either plasma or serum samples will be suitable for clinical applications using microRNAs as cancer diagnostic biomarkers (Mitchell, P.S. et al. (2008) Proc Natl Acad Sci USA 105, 10513-10518; Gilad, S. et al. (2008) PLoS ONE 3, e3148; Chen, X. et al. (2008) Cell Res 18, 997-1006).

Recently, three major studies on serum microRNAs in HCC patients were reported. Qu et al (Qu, KZ. et al (2011) J Clin Gastroenterol 45:355-60) studied the diagnostic potential of serum hsa-miR-16, hsa-miR-195 and hsa-miR-196a on 283 specimens and found that hsa-miR-16 had the best performance with sensitivity of 72.1% and specificity of 88.8%. Xu et al (Xu, J. et al (2011) Molecular Carcinogenesis 50:136-42) found circulating hsa-miR-21, hsa-miR-122 and hsa-miR-223 were potential markers in discriminating HCC from healthy individuals. However those microRNAs can not differentiate HCC from hepatitis B patients. Li et al (Li, LM. et al (2010) Cancer Res 70, 9798-807) reported the outstanding performance of serum microRNA profile - the sensitivity and specificity was 96% and 100% respectively for hsa-miR-375, and was 97.9% and 99.1% for the combination of hsa-miR-375, hsa-miR-25 and hsa-let-7f, respectively. All those results indicated that using serum microRNA profiles in the diagnosis of HCC is feasible, although those studies were limited by one or more of the following factors: limited number of microRNA screened, small sample size, or lack of independent validations. Thus, there remains a need for discovery of diagnostic microRNA biomarkers in plasma or serum of HCC patients. Establishment of blood-based microRNA profiles by combination of multiple microRNA biomarkers will enable a non-invasive, rapid, accurate, and cost-saving identification of patients with early stages of HCC.

### BRIEF SUMMARY OF THE INVENTION

It is a first object of the present invention to provide a novel marker for diagnosing hepatocellular carcinoma, particularly early hepatocellular carcinoma (BCLC stage 0 and A), so as to provide a new approach for diagnosis of hepatocellular carcinoma.

It is a second object of the invention to provide a kit for diagnosing hepatocellular carcinoma, particularly early hepatocellular carcinoma (BCLC stage 0 and A).

It is a third object of the invention to provide a kit for discriminating plasma of hepatocellular carcinoma patients from that of healthy individuals.

It is a forth object of the invention to provide a kit for discriminating plasma of hepatocellular carcinoma patients from that of chronic hepatitis B patients.

It is a fifth object of the invention to provide a kit for discriminating plasma of hepatocellular carcinoma patients from that of cirrhosis patients.

It is a sixth object of the invention to provide a method for determining a marker for diagnosing hepatocellular carcinoma.

These objects as well as others, which will become apparent from the ensuing description, are attained by the subject matter of the independent claims. Some of the preferred embodiments of the present invention are defined by the subject matter of the dependent claims.

In a first aspect, the present invention relates to a marker for diagnosing hepatocellular carcinoma, consisting of a plurality of nucleic acid molecules, each nucleic acid molecule encoding at least one microRNA sequence.

In preferred embodiments, the plurality of nucleic acid molecules comprises at least one nucleic acid molecule encoding at least one microRNA sequence differently expressed in one or more target plasma compared to one or more control plasma.

In more preferred embodiments, the nucleic acid molecule encoding at least one microRNA sequence differently expressed in one or more target plasma compared to one or more controlplasma is selected from at least one nucleic acid molecule encoding at least one microRNA sequence whose expression is up-regulated in one or more target plasma compared to one or more control plasma and at least one nucleic acid molecule encoding at least one microRNA sequence whose expression is down-regulated in one or more target plasma compared to one or more control plasma.

Particularly preferably, the at least one nucleic acid molecule encoding at least one microRNA sequence whose expression is up-regulated in one or more target plasma compared to one or more control plasma is selected from nucleic acid molecules encoding hsa-miR-801, hsa-miR-192 or hsa-miR-21; and the at least one nucleic acid molecule encoding at least one microRNA sequence whose expression is down-regulated in one or more target plasma compared to one or more control plasma is selected from nucleic acid molecules encoding hsa-miR-122, hsa-miR-26a, hsa-miR-27a or hsa-miR-223.

In preferred embodiments, the plurality of nucleic acid molecules also comprises at least one nucleic acid molecule encoding at least one microRNA sequence whose expression is un-changed in one or more target plasma compared to one or more control plasma.

In more preferred embodiments, the nucleic acid molecule encoding at least one microRNA sequence whose expression is un-changed in one or more target plasma compared to one or more control plasma is selected from at least one nucleic acid molecule encoding hsa-miR-1228.

In most preferred embodiments, the plurality of nucleic acid molecules comprises a panel of eight nucleic acid molecules encoding hsa-miR-122, hsa-miR-192, hsa-miR-21, hsa-miR-223, hsa-miR-26a, hsa-miR-27a, hsa-miR-801 and hsa-miR-1228.

In more preferred embodiments, the one or more control plasma are obtained from healthy individuals, chronic hepatitis B patients, or cirrhosis patients.

In preferred embodiments, the hepatocellular carcinoma is early hepatocellular carcinoma.

In a second aspect, the present invention relates to a kit for diagnosing hepatocellular carcinoma, containing a marker for diagnosing hepatocellular carcinoma, wherein the marker for diagnosing hepatocellular carcinoma consists of a plurality of nucleic acid molecules, each nucleic acid molecule encoding at least one microRNA sequence.

In preferred embodiments, the plurality of nucleic acid molecules comprises at least one nucleic acid molecule encoding at least one microRNA sequence differently expressed in one or more target plasma compared to one or more control plasma.

In more preferred embodiments, the nucleic acid molecule encoding at least one microRNA sequence differently expressed in one or more target plasma compared to one or more control plasma is selected from at least one nucleic acid molecule encoding at least one microRNA sequence whose expression is up-regulated in one or more target plasma compared to one or more control plasma and at least one nucleic acid molecule encoding at least one microRNA sequence whose expression is down-regulated in one or more target plasma compared to one or more controlplasma.

Particularly preferably, the at least one nucleic acid molecule encoding at least one microRNA sequence whose expression is up-regulated in one or more target plasma compared to one or more control plasma is selected from nucleic acid molecules encoding hsa-miR-801, hsa-miR-192 or hsa-miR-21; and the at least one nucleic acid molecule encoding at least one microRNA sequence whose expression is down-regulated in one or more target plasma compared to one or more control plasma is selected from nucleic acid molecules encoding hsa-miR-122, hsa-miR-26a, hsa-miR-27a or hsa-miR-223.

In preferred embodiments, the plurality of nucleic acid molecules also comprises at least one nucleic acid molecule encoding at least one microRNA sequence whose expression is un-changed in one or more target plasma compared to one or more control plasma.

In more preferred embodiments, the nucleic acid molecule encoding at least one microRNA sequence whose expression is un-changed in one or more target plasma compared to one or more control plasma is selected from at least one nucleic acid molecule encoding hsa-miR-1228.

In most preferred embodiments, the plurality of nucleic acid molecules comprises a panel of eight nucleic acid molecules encoding hsa-miR-122, hsa-miR-192, hsa-miR-21, hsa-miR-223, hsa-miR-26a, hsa-miR-27a, hsa-miR-801 and hsa-miR-1228. The kit contains also a logistic regression model expressed below: logit(p=HCC)=-1.424-0.292*hsa-miR-122+0.4511*hsa-miR-192+0.6112*hsa-miR-21-0.1796*hsa-miR-223-0.2487*hsa-miR-26a-0.3542*hsa-miR-27a+0.209*hsa-miR-801, wherein the expression levels of hsa-miR-122, hsa-miR-192, hsa-miR-21, hsa-miR-223, hsa-miR-26a, hsa-miR-27a and hsa-miR-801 are detected while using hsa-miR-1228 as endogenous control, and the expression of the logit(p=HCC) value from the model is up-regulated in the one or more target plasma compared to the one or more control plasma.

In more preferred embodiments, the one or more control plasma are obtained from healthy individuals, chronic hepatitis B patients, or cirrhosis patients.

In preferred embodiments, the hepatocellular carcinoma is early hepatocellular carcinoma.

In a third aspect, the present invention relates to a kit for discriminating plasma of at least one hepatocellular carcinoma patient from that of at least one healthy individuals, containing a marker for diagnosing hepatocellular carcinoma, wherein the marker for diagnosing hepatocellular carcinoma consists of a plurality of nucleic acid molecules, each nucleic acid molecule encoding at least one microRNA sequence, the plurality of nucleic acid molecules comprises at least one nucleic acid molecule encoding the microRNA sequence differently expressed in one or more target plasma compared to one or more control plasma, and the control plasma are obtained from healthy individuals.

In preferred embodiments, the plurality of nucleic acid molecules comprises at least one nucleic acid molecule encoding at least one microRNA sequence differently expressed in one or more target plasma compared to one or more control plasma.

In more preferred embodiments, the nucleic acid molecule encoding at least one microRNA sequence differently expressed in one or more target plasma compared to one or more control plasma is selected from at least one nucleic acid molecule encoding at least one microRNA sequence whose expression is up-regulated in one or more target plasma compared to one or more control plasma and at least one nucleic acid molecule encoding at least one microRNA sequence whose expression is down-regulated in one or more target plasma compared to one or more control plasma.

Particularly preferably, the at least one nucleic acid molecule encoding at least one microRNA sequence whose expression is up-regulated in one or more target plasma compared to one or more control plasma is selected from nucleic acid molecules encoding hsa-miR-801, hsa-miR-192 or hsa-miR-21; and the at least one nucleic acid molecule encoding at least one microRNA sequence whose expression is down-regulated in one or more target plasma compared to one or more control plasma is selected from nucleic acid molecules encoding hsa-miR-122, hsa-miR-26a, hsa-miR-27a or hsa-miR-223.

In preferred embodiments, the plurality of nucleic acid molecules also comprises at least one nucleic acid molecule encoding at least one microRNA sequence whose expression is un-changed in one or more target plasma compared to one or more control plasma.

In more preferred embodiments, the nucleic acid molecule encoding at least one microRNA sequence whose expression is un-changed in one or more target plasma compared to one or more control plasma is selected from at least one nucleic acid molecule encoding hsa-miR-1228.

In most preferred embodiments, the plurality of nucleic acid molecules comprises a panel of eight nucleic acid molecules encoding hsa-miR-122, hsa-miR-192, hsa-miR-21, hsa-miR-223, hsa-miR-26a, hsa-miR-27a, hsa-miR-801 and hsa-miR-1228. The kit contains also a logistic regression model expressed below: logit(p=HCC)=-1.424-0.292*hsa-miR-122+0.4511*hsa-miR-192+0.6112*hsa-miR-21-0.1796*hsa-miR-223-0.2487*hsa-miR-26a-0.3542*hsa-miR-27a+0.209*hsa-miR-801, wherein the expression levels of hsa-miR-122, hsa-miR-192, hsa-miR-21, hsa-miR-223, hsa-miR-26a, hsa-miR-27a and hsa-miR-801 are detected while using hsa-miR-1228 as endogenous control, and the expression of the logit(p=HCC) value from the model is up-regulated in the one or more target plasma compared to the one or more control plasma.

In preferred embodiments, the hepatocellular carcinoma is early hepatocellular carcinoma.

In a forth aspect, the present invention relates to a kit for discriminating plasma of at least one hepatocellular carcinoma patient from that of at least one chronic hepatitis B patients, containing a marker for diagnosing hepatocellular carcinoma, wherein the marker for diagnosing hepatocellular carcinoma consists of a plurality of nucleic acid molecules, each nucleic acid molecule encoding at least one microRNA sequence, the plurality of nucleic acid molecules comprises at least one nucleic acid molecule encoding the microRNA sequence differently expressed in one or more target plasma compared to one or more control plasma, and the control plasma are obtained from chronic hepatitis B patients.

In preferred embodiments, the plurality of nucleic acid molecules comprises at least one nucleic acid molecule encoding at least one microRNA sequence differently expressed in one or more target plasma compared to one or more control plasma.

In more preferred embodiments, the nucleic acid molecule encoding at least one microRNA sequence differently expressed in one or more target plasma compared to one or more controlplasma is selected from at least one nucleic acid molecule encoding at least one microRNA sequence whose expression is up-regulated in one or more target plasma compared to one or more control plasma and at least one nucleic acid molecule encoding at least one microRNA sequence whose expression is down-regulated in one or more target plasma compared to one or more control plasma.

Particularly preferably, the at least one nucleic acid molecule encoding at least one microRNA sequence whose expression is up-regulated in one or more target plasma compared to one or more control plasma is selected from nucleic acid molecules encoding hsa-miR-801, hsa-miR-192 or hsa-miR-21; and the at least one nucleic acid molecule encoding at least one microRNA sequence whose expression is down-regulated in one or more target plasma compared to one or more control plasma is selected from nucleic acid molecules encoding hsa-miR-122, hsa-miR-26a, hsa-miR-27a or hsa-miR-223.

In preferred embodiments, the plurality of nucleic acid molecules also comprises at least one nucleic acid molecule encoding at least one microRNA sequence whose expression is un-changed in one or more target plasma compared to one or more control plasma.

In more preferred embodiments, the nucleic acid molecule encoding at least one microRNA sequence whose expression is un-changed in one or more target plasma compared to one or more control plasma is selected from at least one nucleic acid molecule encoding hsa-miR-1228.

In most preferred embodiments, the plurality of nucleic acid molecules comprises a panel of eight nucleic acid molecules encoding hsa-miR-122, hsa-miR-192, hsa-miR-21, hsa-miR-223, hsa-miR-26a, hsa-miR-27a, hsa-miR-801 and hsa-miR-1228. The kit contains also a logistic regression model expressed below: logit(p=HCC)=-1.424-0.292*hsa-miR-122+0.4511*hsa-miR-192+0.6112*hsa-miR-21-0.1796*hsa-miR-223-0.2487*hsa-miR-26a-0.3542*hsa-miR-27a+0.209*hsa-miR-801, wherein the expression levels of hsa-miR-122, hsa-miR-192, hsa-miR-21, hsa-miR-223, hsa-miR-26a, hsa-miR-27a and hsa-miR-801 are detected while using hsa-miR-1228 as endogenous control, and the expression of the logit(p=HCC) value from the model is up-regulated in the one or more target plasma compared to the one or more control plasma.

In preferred embodiments, the hepatocellular carcinoma is early hepatocellular carcinoma.

In a fifth aspect, the present invention relates to a kit for discriminating plasma of at least one hepatocellular carcinoma patient from that of at least one cirrhosis patients, containing a marker for diagnosing hepatocellular carcinoma, wherein the marker for diagnosing hepatocellular carcinoma consists of a plurality of nucleic acid molecules, each nucleic acid molecule encoding at least one microRNA sequence, the plurality of nucleic acid molecules comprises at least one nucleic acid molecule encoding the microRNA sequence differently expressed in one or more target plasma compared to one or more control plasma, and the control plasma are obtained from cirrhosis patients.

In preferred embodiments, the plurality of nucleic acid molecules comprises at least one nucleic acid molecule encoding at least one microRNA sequence differently expressed in one or more target plasma compared to one or more control plasma.

In more preferred embodiments, the nucleic acid molecule encoding at least one microRNA sequence differently expressed in one or more target plasma compared to one or more controlplasma is selected from at least one nucleic acid molecule encoding at least one microRNA sequence whose expression is up-regulated in one or more target plasma compared to one or more control plasma and at least one nucleic acid molecule encoding at least one microRNA sequence whose expression is down-regulated in one or more target plasma compared to one or more control plasma.

Particularly preferably, the at least one nucleic acid molecule encoding at least one microRNA sequence whose expression is up-regulated in one or more target plasma compared to one or more control plasma is selected from nucleic acid molecules encoding hsa-miR-801, hsa-miR-192 or hsa-miR-21; and the at least one nucleic acid molecule encoding at least one microRNA sequence whose expression is down-regulated in one or more target plasma compared to one or more control plasma is selected from nucleic acid molecules encoding hsa-miR-122, hsa-miR-26a, hsa-miR-27a or hsa-miR-223.

In preferred embodiments, the plurality of nucleic acid molecules also comprises at least one nucleic acid molecule encoding at least one microRNA sequence whose expression is un-changed in one or more target plasma compared to one or more control plasma.

In more preferred embodiments, the nucleic acid molecule encoding at least one microRNA sequence whose expression is un-changed in one or more target plasma compared to one or more control plasma is selected from at least one nucleic acid molecule encoding hsa-miR-1228.

In most preferred embodiments, the plurality of nucleic acid molecules comprises a panel of eight nucleic acid molecules encoding hsa-miR-122, hsa-miR-192, hsa-miR-21, hsa-miR-223, hsa-miR-26a, hsa-miR-27a, hsa-miR-801 and hsa-miR-1228. The kit contains also a logistic regression model expressed below: logit(p=HCC)=-1.424-0.292*hsa-miR-122+0.4511*hsa-miR-192+0.6112*hsa-miR-21-0,1796*hsa-miR-223-0.2487*hsa-miR-26a-0.3542*hsa-miR-27a+0.209*hsa-miR-801, wherein the expression levels of hsa-miR-122, hsa-miR-192, hsa-miR-21, hsa-miR-223, hsa-miR-26a, hsa-miR-27a and hsa-miR-801 are detected while using hsa-miR-1228 as endogenous control, and the expression of the logit(p=HCC) value from the model is up-regulated in the one or more target plasma compared to the one or more control plasma.

In preferred embodiments, the hepatocellular carcinoma is early hepatocellular carcinoma.

In a sixth aspect, the present invention relates to a method for determining a marker for diagnosing hepatocellular carcinoma, comprising:
(a) determining in one or more target plasma the expression levels of a plurality of nucleic acid molecules, each nucleic acid molecule encoding at least one microRNA sequence;
(b) determining in one or more control plasma the expression levels of the plurality of nucleic acid molecules; and
(c) identifying from the plurality of nucleic acid molecules one or more nucleic acid molecules that are differentially expressed in the target plasma and the control plasma by comparing the respective expression levels obtained in steps (a) and (b) from the plurality of nucleic acid molecules, and identifying one or more target plasma exhibiting hepatocellular carcinoma by using the one or more nucleic acid molecules that are differentially expressed in the target plasma and the control plasma as a marker for diagnosing hepatocellular carcinoma.

Other embodiments of the present invention will become apparent from the detailed description hereinafter.

### BRIEF DESCRIPTION OF THE DRAWINGS

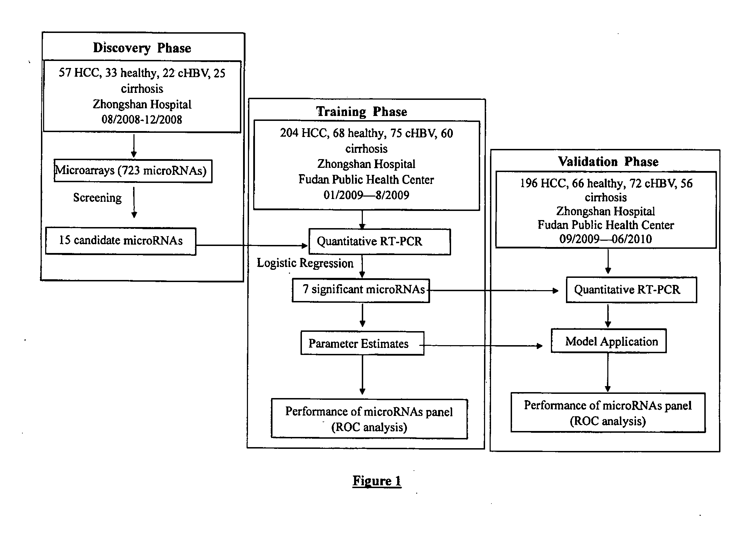

Figure 1 depicts a flow chart schematically illustrating the study design in the discovery, training and validation phases of a microRNA panel according to the present invention for identifying one or more target plasma exhibiting hepatocellular carcinoma, particularly for the presence of early hepatocellular carcinoma (BCLC stage 0 and A).

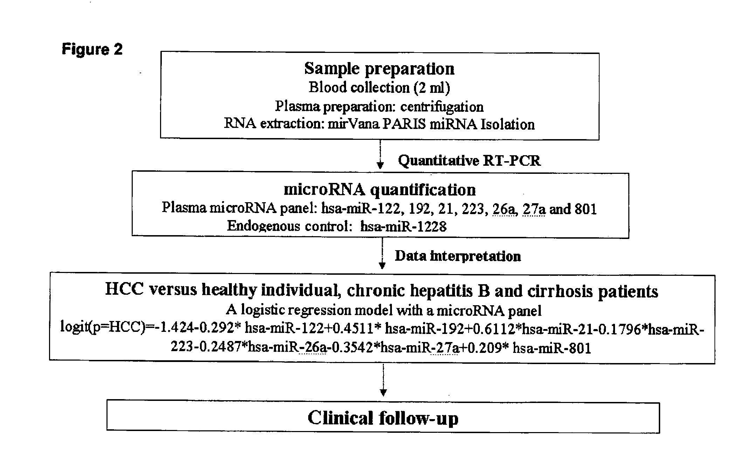

Figure 2 depicts a flow chart schematically illustrating the essential method steps for determining a microRNA panel in blood according to the present invention for diagnosing hepatocellular carcinoma, particularly for diagnosing early hepatocellular carcinoma (BCLC stage 0 and A). The control group included healthy, chronic hepatitis B and cirrhosis subjects.

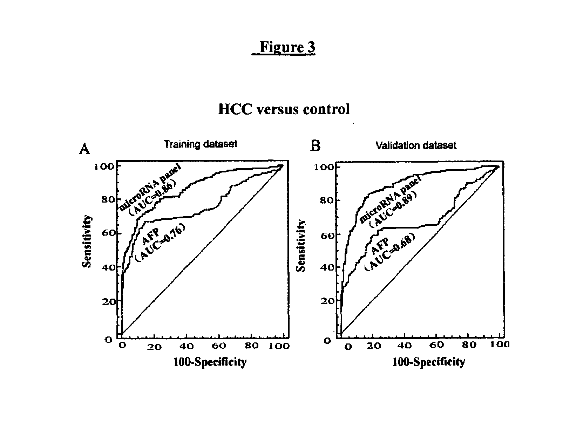

Figure 3 illustrates the logistic regression model comprising preferred a microRNA panel (hsa-miR-122, hsa-miR-192, hsa-miR-21, hsa-miR-223, hsa-miR-26a, hsa-miR-27a and hsa-miR-801) according to the present invention for identifying one or more target plasma exhibiting hepatocellular carcinoma. A) ROC plot for the logit value of the microRNA panel in the training dataset (n=407) for HCC group versus the control. Comparing to AFP (AUC=0.76), the microRNA panel had significantly higher diagnostic accuracy (AUC=0.86) in discriminating biological samples of HCC patients from those of the control. B) ROC plot for the logit value of the microRNA panel in the validation dataset (n=390) for HCC versus the control. Comparing to AFP (AUC=0.68), the microRNA panel had significantly higher diagnostic accuracy (AUC=0.89) in discriminating HCC from the control.

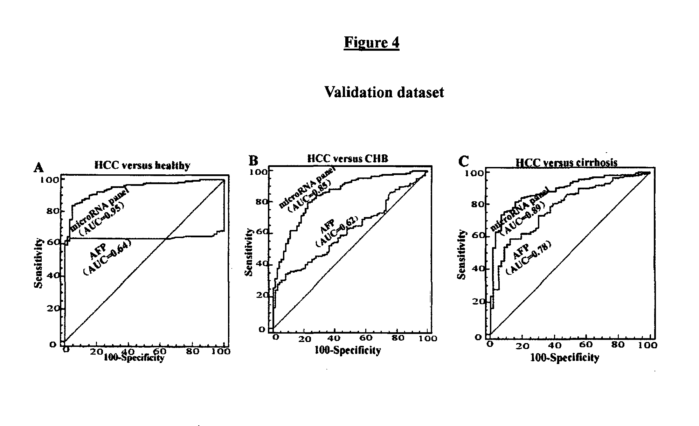

Figure 4 illustrates the logistic regression model comprising preferred a microRNA panel (hsa-miR-122, hsa-miR-192, hsa-miR-21, hsa-miR-223, hsa-miR-26a, hsa-miR-27a and hsa-miR-801) according to the present invention for further discriminating biological samples of hepatocellular carcinoma from those of healthy individuals, chronic hepatitis B patients, or cirrhosis patients. A) ROC plot for the logit value of the microRNA panel in the validation dataset (n=390) for HCC group versus the healthy group. Comparing to AFP (AUC=0.64), the microRNA panel had significantly higher diagnostic accuracy (AUC=0.95) in discriminating biological samples of HCC from those of healthy individuals. B) ROC plot for the logit value of the microRNA panel in the validation dataset (n=390) for HCC versus chronic hepatitis B patients. Comparing to AFP (AUC=0.62), the microRNA panel had significantly higher diagnostic accuracy (AUC=0.85) in discriminating biological samples of HCC from those of chronic hepatitis B patients. C) ROC plot for the logit value of the microRNA panel in the validation dataset (n=390) for HCC versus cirrhosis patients. Comparing to AFP (AUC=0.78), the microRNA panel had significantly higher diagnostic accuracy (AUC=0.89) in discriminating biological samples of HCC from those of cirrhosis patients.

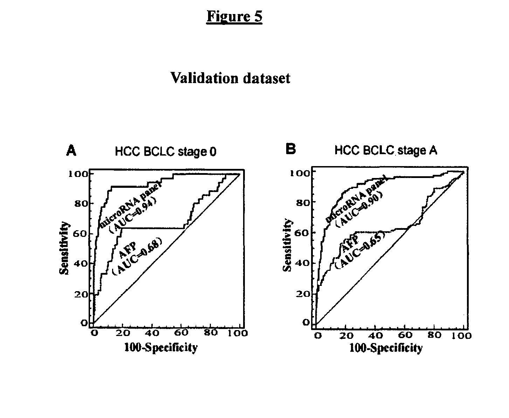

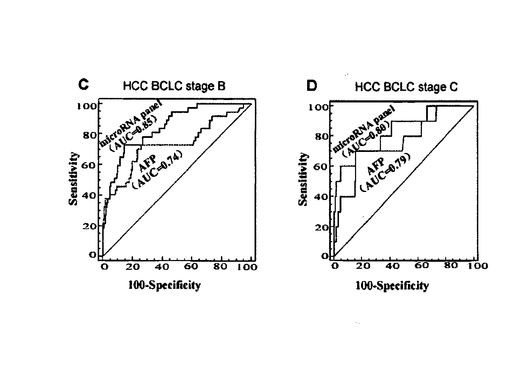

Figure 5 illustrates the logistic regression model comprising preferred a microRNA panel (hsa-miR-122, hsa-miR-192, hsa-miR-21, hsa-miR-223, hsa-miR-26a, hsa-miR-27a and hsa-miR-801) according to the present invention for identifying one or more target plasma exhibiting different BCLC stages of hepatocellular carcinoma. A) ROC plot for the logit value of the microRNA panel on very early stage HCC (BCLC 0) versus the control. Comparing to AFP (AUC=0.68), the microRNA panel had much higher diagnostic accuracy (AUC=0.94) in discriminating very early stage HCC from the control. B) ROC plot for the logit value of the microRNA panel on early stage HCC (BCLC A) versus the control. Comparing to AFP (AUC=0.65), the microRNA panel had much higher diagnostic accuracy (AUC=0.90) in discriminating early stage HCC from the control. C) ROC plot for the logit value of the microRNA panel on intermediate stage HCC (BCLC B) versus the control. Comparing to AFP (AUC=0.74), the microRNA panel had much higher diagnostic accuracy (AUC=0.85) in discriminating intermediate stage HCC from the control. D) ROC plot for the logit value of the microRNA panel on advanced stage HCC (BCLC C) versus the control. Comparing to AFP (AUC=0.79), the microRNA panel (AUC=0.80) had no significant difference in discriminating advanced stage HCC from the control.

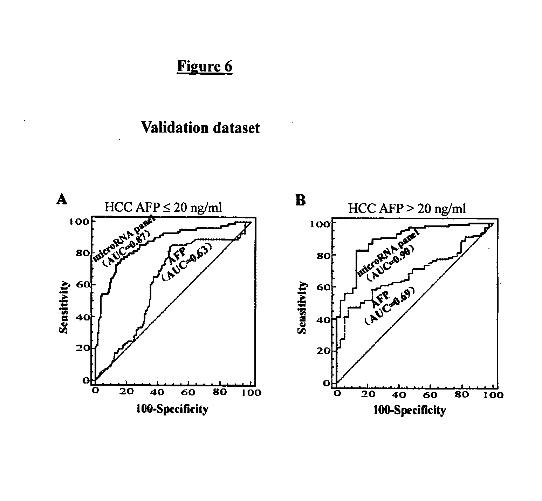

Figure 6 illustrates the logistic regression model comprising preferred a microRNA panel (hsa-miR-122, hsa-miR-192, hsa-miR-21, hsa-miR-223, hsa-miR-26a, hsa-miR-27a and hsa-miR-801) according to the present invention for identifying one or more target plasma exhibiting hepatocellular carcinoma with AFP ≤20 ng/ml and AFP > 20 ng/ml. A) ROC plot for the logit value of the microRNA panel on HCC cases with AFP ≤ 20 ng/ml versus the control. Comparing to AFP (AUC=0.63), the microRNA biomarkers had much higher diagnostic accuracy (AUC=0.87) in discriminating the HCC cases with AFP ≤20 ng/ml from the control. B) ROC plot for the logit value of the microRNA panel on HCC cases with AFP >20 ng/ml versus the control Comparing to AFP (AUC=0.69), the microRNA biomarkers had much higher diagnostic accuracy (AUC=0.90) in discriminating the HCC cases with AFP >20 ng/ml from the control.

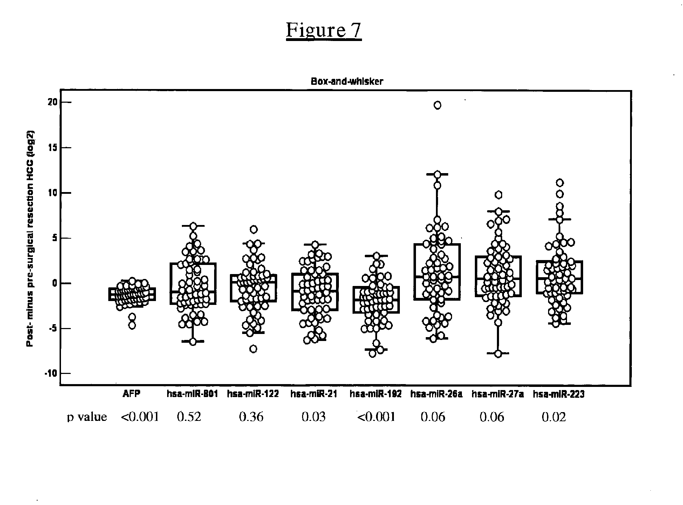

Figure 7 depicts Box-and-whisker plot on the perioperative change of 7 selected microRNAs and AFP. Blood from 54 HCC patients, who received surgical liver resections were obtained both preoperatively and the 6^{th} days post-operatively. The expression level of AFP and 3 microRNAs (hsa-miR-21, hsa-miR-192 and hsa-miR-223) had significant changes after the HCC resections on 6^{th} days. The expression levels of AFP and 2 miRNAs (hsa-miR-21 and hsa-miR-192) significantly reduced, while the expression of hsa-miR-223 significantly increased after the surgical resections.

### DETAILED DESCRIPTION OF THE INVENTION

The present invention is based on the unexpected finding that hepatocellular carcinoma can be reliably identified based on a marker for diagnosing hepatocellular carcinoma with high diagnostic accuracy, wherein the marker for diagnosing hepatocellular carcinoma as defined herein typically comprises both up- and down-regulated human microRNAs. More specifically, said marker for diagnosing hepatocellular carcinoma - by analyzing the overall microRNA expression pattern and/or the respective individual microRNA expression level(s) in plasma - allow the detection of hepatocellular carcinoma at a very early disease state and discrimination of plasma of hepatocellular carcinoma patients from that of healthy individuals, chronic hepatitis B patients and cirrhosis patients.

The present invention illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims. The drawings described are only schematic and are to be considered nonlimiting.

Where the term "comprising" is used in the present description and claims, it does not exclude other elements or steps. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun e.g. "a" or "an", "the", this includes a plural of that noun unless something else is specifically stated.

The term "about" in the context of the present invention denotes an interval of accuracy that the person skilled in the art will understand to still ensure the technical effect of the feature in question. The term typically indicates deviation from the indicated numerical value of ± 10%, and preferably ± 5%.

Furthermore, the terms first, second, third, (a), (b), (c), and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context which the terms are used.

The following terms or definitions are provided solely to aid in the understanding of the invention. These definitions should not be construed to have a scope less than understood by a person of ordinary skill in the art.

The term "cancer" (also referred to as "carcinoma"), as used herein, generally denotes any type of malignant neoplasm, that is, any morphological and/or physiological alterations (based on genetic re-programming) of target cells exhibiting or having a predisposition to develop characteristics of a carcinoma as compared to unaffected (healthy) wild-type control cells. Examples of such alterations may relate inter alia to cell size and shape (enlargement or reduction), cell proliferation (increase in cell number), cell differentiation (change in physiological state), apoptosis (programmed cell death) or cell survival

The term "hepatocellular", as used herein, relates to cells of the liver. Hence, the term "hepatocellular cancer" refers to cancerous growths in the liver.

The most common type of liver cancer is hepatocellular carcinoma (also referred to as "hepatoma" and commonly abbreviated as "HCC"). The term "hepatocellular carcinoma", as used herein, denotes a primary malignancy of the liver. Most cases of HCC are secondary to either a viral hepatitide infection (hepatitis B or C) or cirrhosis (alcoholism being the most common cause of hepatic cirrhosis). In countries where hepatitis is not endemic, most malignant cancers in the liver are not primary HCC but metastasis (spread) of cancer from elsewhere in the body, e.g. the colon. Treatment options of HCC and prognosis are dependent on many factors but especially on tumor size and staging. The usual outcome is poor, because only 10% to 20% of hepatocellular carcinomas can be removed completely using surgery. If the cancer cannot be completely removed, the disease is usually deadly within 3 to 6 months.

Hepatocellular carcinoma, like any other cancer, develops when there is a mutation to the cellular machinery that causes the cell to replicate at a higher rate and/or results in the cell avoiding apoptosis. In particular, chronic viral infections of hepatitis B and/or C can aid the development of hepatocellular carcinoma by repeatedly causing the body's own immune system to attack the liver cells, some of which are infected by the virus, others merely bystanders. While this constant cycle of damage followed by repair can lead to mistakes during repair which in turn lead to carcinogenesis, this hypothesis is more applicable, at present, to hepatitis C. In hepatitis B, however, the integration of the viral genome into infected cells is the most consistently associated factor in malignancy. Alternatively, repeated consumption of large amounts of ethanol can have a similar effect.

Barcelona Clinic Liver Cancer (BCLC) staging classification comprises five stages. Very early stage (0) includes patients with asymptomatic single HCC < 2 cm Early stage (A) includes patients with asymptomatic single or three HCC <= 3 cm. Intermediate stage (B) includes patients with asymptomatic multinodular HCC. Advanced stage (C) includes patients with symptomatic tumors and/or an invasive tumoral pattern (vascular invasion/extrahepatic spread). End-stage disease (D) includes patients with extremely grim prognosis.

Thus, within the scope of the present invention hepatitis B infections or hepatic cirrhosis are not merely to be considered as risk factors for tumor etiology but as early/intermediate stages in tumor progression (i.e. "pre-cancerous states") that are associated with hyper-proliferative tissue growth resulting in (often benign) non-invasive neoplasm which, in turn, may progress to malignant tumors such as HCC.

Such malignant tumors invade other tissues and often metastasize given enough time to do so. Malignant cells are often characterized by progressive and uncontrolled growth. Macroscopically, HCC appears as a nodular or infiltrative tumor. The nodular type may be solitary (having a large mass) or multiple (when developed as a complication of cirrhosis). Tumor nodules are round to oval, well circumscribed but not encapsulated. The diffuse type is poorly circumscribed and infiltrates the portal veins, rarely the hepatic veins.

The mammalian target plasma employed in the present invention may be of human or non-human origin. However, the invention is typically performed with human plasma. The term "one or more plasma", as used herein, is to be understood not only to include individual plasma. The term "target plasma", as used herein, refers to plasma being at least supposed to exhibit hepatocellular carcinoma, whereas the term "control plasma" typically denotes plasma obtained from healthy individual, chronic hepatitis B and cirrhosis patients not having characteristics of such a cancerous phenotype. However, in some applications, for example, when comparing plasma exhibiting different cancer types, the plasma not having characteristics of such a hepatocellular cancerous phenotype are typically considered the "control plasma".

The term "plasma", as used herein, is the yellow liquid component of blood, in which the blood cells in whole blood would normally be suspended. It makes up about 55% of the total blood volume. It is mostly water (90% by volume) and contains dissolved proteins, glucose, clotting factors, mineral ions, hormones and carbon dioxide (plasma being the main medium for excretory product transportation). Plasma is prepared by spinning a tube of fresh blood in a centrifuge until the blood cells fall to the bottom of the tube. The plasma is then poured or drawn off. Plasma has a density of approximately 1025 kg/m³, or 1.025 kg/l. Recent research showed that microRNA is stable in plasma. The term "plasma sample" refers to plasma taken from individuals being examined or from control.

The term "patient", as used herein, refers to a human being at least supposed to have hepatocellular carcinoma where as "target plasma", as used herein, refers to plasma collected from patients; The term "healthy individual" typically denotes a healthy person not having characteristics of such a cancerous phenotype. And "control plasma", as used herein, denotes plasma collected from healthy individuals, chronic hepatitis B patients, and cirrhosis patients. However, in some applications, for example, when comparing different cancer types, the individual having the other cancer types and plasma collected from these individuals is typically considered the "control".

Typically, the plasma samples used are derived from biological specimens collected from the subjects to be diagnosed for the presence of hepatocellular carcinoma. Furthermore, in order to corroborate the data obtained, "comparative samples" may also be collected from subjects having a given known disease state. The biological samples may include body tissues and fluids, such as tissue, serum, blood cell, sputum, and urine. Furthermore, the biological sample may be obtained from individual having hepatocellular cancerous characteristics or suspected to be cancerous. Furthermore, the sample may be purified from the obtained body tissues and fluids if necessary, and then used as the biological sample. According to the present invention, the expression level of the nucleic acid markers of the present invention is determined in the subject-derived biological sample(s).

The sample used for detection in the in vitro methods of the present invention should generally be collected in a clinically acceptable manner, preferably in a way that nucleic acids (in particular RNA) or proteins are preserved. The samples to be analyzed are typically from blood. Furthermore, liver tissue and other types of sample can be used as well. Samples, in particular after initial processing may be pooled. However, also non-pooled samples may be used.

The term "microRNA" (or "miRNA"), as used herein, is given its ordinary meaning in the art (Bartel, D.P. (2004) Cell 23, 281-292; He, L. and Hannon, G.J. (2004) Nat Rev Genet 5, 522-531). Accordingly, a "microRNA" denotes an RNA molecule derived from a genomic locus that is processed from transcripts that can form local RNA precursor miRNA structures. The mature miRNA is usually 20, 21, 22, 23, 24, or 25 nucleotides in length, although other numbers of nucleotides may be present as well, for example 18, 19, 26 or 27 nucleotides.

The miRNA encoding sequence has the potential to pair with flanking genomic sequences, placing the mature miRNA within an imperfect RNA duplex (herein also referred to as stem-loop or hairpin structure or as pre-miRNA), which serves as an intermediate for miRNA processing from a longer precursor transcript. This processing typically occurs through the consecutive action of two specific endonucleases termed Drosha and Dicer, respectively. Drosha generates from the primary transcript (herein also denoted "pri-miRNA") a miRNA precursor (herein also denoted "pre-miRNA") that typically folds into a hairpin or stem-loop structure. From this miRNA precursor a miRNA duplex is excised by means of Dicer that comprises the mature miRNA at one arm of the hairpin or stem-loop structure and a similar-sized segment (commonly referred to miRNA*) at the other arm. The miRNA is then guided to its target mRNA to exert its function, whereas the miRNA* is degraded. In addition, miRNAs are typically derived from a segment of the genome that is distinct from predicted protein-coding regions.

The term "miRNA precursor" (or "precursor miRNA" or "pre-miRNA"), as used herein, refers to the portion of a miRNA primary transcript from which the mature miRNA is processed. Typically, the pre-miRNA folds into a stable hairpin (i.e. a duplex) or a stem-loop structure. The hairpin structures typically range from 50 to 80 nucleotides in length, preferably from 60 to 70 nucleotides (counting the miRNA residues, those pairing to the miRNA, and any intervening segment(s) but excluding more distal sequences).

The term "nucleic acid molecule encoding a microRNA sequence", as used herein, denotes any nucleic acid molecule coding for a microRNA (miRNA). Thus, the term does not only refer to mature miRNAs but also to the respective precursor miRNAs and primary miRNA transcripts as defined above. Furthermore, the present invention is not restricted to RNA molecules but also includes corresponding DNA molecules encoding a microRNA, e.g. DNA molecules generated by reverse transcribing a miRNA sequence. A nucleic acid molecule encoding at least one microRNA sequence according to the invention typically encodes a single miRNA sequence (i.e. an individual miRNA). However, it is also possible that such nucleic acid molecule encodes two or more miRNA sequences (i.e. two or more miRNAs), for example a transcriptional unit comprising two or more miRNA sequences under the control of common regulatory sequences such as a promoter or a transcriptional terminator.

The term "nucleic acid molecule encoding a microRNA sequence", as used herein, is also to be understood to include "sense nucleic acid molecules" (i.e. molecules whose nucleic acid sequence (5'→ 3') matches or corresponds to the encoded miRNA (5'→ 3') sequence) and "anti-sense nucleic acid molecules" (i.e. molecules whose nucleic acid sequence is complementary to the encoded miRNA (5'→ 3') sequence or, in other words, matches the reverse complement (3'→ 5') of the encoded miRNA sequence). The term "complementary", as used herein, refers to the capability of an "anti-sense" nucleic acid molecule sequence of forming base pairs, preferably Watson-Crick base pairs, with the corresponding "sense" nucleic acid molecule sequence (having a sequence complementary to the anti-sense sequence).

Within the scope of the present invention, two nucleic acid molecules (i.e. the "sense" and the "anti-sense" molecule) may be perfectly complementary, that is, they do not contain any base mismatches and/or additional or missing nucleotides. Alternatively, the two molecules comprise one or more base mismatches or differ in their total numbers of nucleotides (due to additions or deletions). Preferably, the "complementary" nucleic acid molecule comprises at least ten contiguous nucleotides showing perfect complementarity with a sequence comprised in corresponding "sense" nucleic acid molecule.

Accordingly, the plurality of nucleic acid molecules encoding a miRNA sequence that are comprised in a diagnostic kit of the present invention may include one or more "sense nucleic acid molecules" and/or one or more "anti-sense nucleic acid molecules". In case, the diagnostic kit includes one or more "sense nucleic acid molecules" (i.e. the miRNA sequences as such), said molecules are to be considered to constitute the totality or at least a subset of differentially expressed miRNAs (i.e. molecular markers) being indicative for the presence of or the disposition to develop a particular condition, here hepatocellular cancer. On the other hand, in case a diagnostic kit includes one or more "anti-sense nucleic acid molecules" (i.e. sequences complementary to the miRNA sequences), said molecules may comprise inter alia probe molecules (for performing hybridization assays) and/or oligonucleotide primers (e.g., for reverse transcription or PCR applications) that are suitable for detecting and/or quantifying one or more particular (complementary) miRNA sequences in a given sample.

A plurality of nucleic acid molecules as defined within the present invention may comprise at least two, at least ten, at least 50, at least 100, at least 200, at least 500, at least 1.000, at least 10.000 or at least 100.000 nucleic acid molecules, each molecule encoding at least one miRNA sequence.

The term "differentially expressed", as used herein, denotes an altered expression level of a particular microRNA in the target plasma as compared to in the plasma control which is plasma obtained from healthy individuals or patients with other types of disease, which may be an up-regulation (i.e. an increased microRNAs concentration in the plasma) or a down-regulation (i.e. a reduced or abolished microRNA concentration in the plasma). In other words, the nucleic acid molecule is activated to a higher or lower level in the disease plasma samples than in the control plasma.

Within the scope of the present invention, a nucleic acid molecule is to considered differentially expressed if the respective expression levels of this nucleic acid molecule in disease plasma samples and control samples typically differ by at least 5% or at least 10%, preferably by at least 20% or at least 25%, and most preferably by at least 30% or at least 50%. Thus, the latter values correspond to an at least 1.3-fold or at least 1.5-fold up-regulation of the expression level of a given nucleic acid molecule in the target plasma samples compared to the controlplasma samples or vice versa an at least 0.7-fold or at least 0.5-fold down-regulation of the expression level in the target plasma samples, respectively.

The term "expression level", as used herein, refers to extent to which a particular miRNA sequence is transcribed from its genomic locus, that is, the concentration of a miRNA in the plasma sample to be analyzed.

As outlined above, the term "control plasma" typically denotes a plasma sample collected from individual not having characteristics of a hepatocellular cancer phenotype. However, in some applications, for example, when comparing other cancer types, the plasma collected from the patients with other cancer types is typically considered the "control plasma".

The determining of expression levels typically follows established standard procedures well known in the art (Sambrook, J. et al. (1989) Molecular Cloning: A Laboratory Manual. 2nd Ed., Cold Spring Harbor Laboratory Press, Cold Spring Harbor, NY; Ausubel, F.M. et al. (2001) Current Protocols in Molecular Biology. Wiley & Sons, Hoboken, NJ). Determination may occur at the RNA level, for example by Northern blot analysis using miRNA-specific probes, or at the DNA level following reverse transcription (and cloning) of the RNA population, for example by quantitative PCR or real-time PCR techniques. The term "determining", as used herein, includes the analysis of any nucleic acid molecules encoding at least one microRNA sequence as described above. However, due to the short half-life of pri-miRNAs and pre-mRNAs typically the concentration of only the mature miRNA is measured.

In specific embodiments, the standard value of the expression levels obtained in several independent measurements of a given sample (for example, two, three, five or ten measurements) and/or several measurements within several target samples or control samples are used for analysis. The standard value may be obtained by any method known in the art. For example, a range of mean ± 2 SD (standard deviation) or mean ± 3 SD may be used as standard value.

The difference between the expression levels obtained for target and control plasma may be normalized to the expression level of further control nucleic acids, e.g. housekeeping genes whose expression levels are known not to differ depending on the disease states of the individual from whom the sample was collected. Exemplary housekeeping genes include inter alia β-actin, glycerinaldehyde 3-phosphate dehydrogenase, and ribosomal protein P1. In preferred embodiments, the control nucleic acid is another miRNA known to be stably expressed during the various non-cancerous and (pre-)cancerous states of the individual from whom the sample was collected.

However, instead of determining in any experiment the expression levels for plasma sample it may also be possible to define based on experimental evidence and/or prior art data one or more cut-off values for a particular disease phenotype (i.e. a disease state). In such scenario, the respective expression levels for the plasma sample can be determined by using a stably expressed control microRNA for normalization. If the "normalized" expression levels calculated are higher than the respective cutoff value defined, then this finding would be indicative for an up-regulation of gene expression. Vice versa, if the "normalized" expression levels calculated are lower than the respective cutoff value defined, then this finding would be indicative for a down-regulation of microRNA expression.

In the context of the present invention, the term "identifying hepatocellular carcinoma and/or discriminating other HBV infection related diseases" is intended to also encompass predictions and likelihood analysis (in the sense of "diagnosing"). The compositions and methods disclosed herein are intended to be used clinically in making decisions concerning treatment modalities, including therapeutic intervention, diagnostic criteria such as disease stages, and disease monitoring and surveillance for the disease. According to the present invention, an intermediate result for examining the condition of a subject may be provided. Such intermediate result may be combined with additional information to assist a doctor, nurse, or other practitioner to diagnose that a subject suffers from the disease. Alternatively, the invention may be used to detect cancerous changes through plasma sample, and provide a doctor with useful information for diagnosis. Furthermore, the invention may also be used to discriminate between hepatocellular carcinoma and other HBV infection related diseases including chronic hepatitis B and cirrhosis.

Within the present invention, one or more differentially expressed nucleic acid molecules identified together represent a marker that is indicative for hepatocellular carcinoma through plasma sample. The term "marker", as used herein, denotes a set of nucleic acid molecules (e.g., miRNAs), wherein the expression level of the individual nucleic acid molecules differs between the plasma collected from hepatocellular carcinoma patient and the control plasma. Herein, a marker is also referred to as a set of markers and represents a minimum number of (different) nucleic acid molecules, each encoding at least one miRNA sequence that is capable for identifying a phenotypic state of an individual

In a first aspect, the present invention relates to a marker for diagnosing hepatocellular carcinoma, consisting of a plurality of nucleic acid molecules, each nucleic acid molecule encoding at least one microRNA sequence.

Typically, the nucleic acid molecules comprised in the marker for diagnosing hepatocellular carcinoma are human sequences (hereinafter designated "hsa" (Homo sapiens).

Particularly preferably, the plurality of nucleic acid molecules comprises one or more of the nucleic acid molecules encoding hsa-miR-122 (SEQ ID NO:1), hsa-miR-192 (SEQ ID NO:2), hsa-miR-21 (SEQ ID NO:3), hsa-miR-223 (SEQ ID NO:4), hsa-miR-26a (SEQ ID NO:5), hsa-miR-27a (SEQ ID NO:6), hsa-miR-801(SEQ ID NO:7) and an endogenous control hsa-miR-1228 (SEQ ID NO:8).

The nucleic acid sequences of the above-referenced microRNAs are listed in Table 1.

**TABLE 1**

| microRNA | Sequence (5' → 3') |
|---|---|
| hsa-miR-122 | uggagugugacaaugguguuug |
| hsa-miR-192 | cugaccuaugaauugacagcc |
| hsa-miR-21 | uagcuuaucagacugauguuga |
| hsa-miR-223 | ugucaguuugucaaauacccca |
| hsa-miR-26a | uucaaguaauccaggauaggcu |
| hsa-miR-27a | uucacaguggcuaaguuccgc |
| hsa-miR-801 | gauugcucugcgugcggaaucgac |
| hsa-miR-1228 | ucacaccugccucgcccccc |

All microRNA sequences disclosed herein have been deposited in the miRBase database (http://microrna.sanger.ac.uk/; see also Griffiths-Jones S. et al. (2008) Nucl. Acids Res. 36, D154-D158).

The expression of any one or more of the nucleic acid molecules encoding hsa-miR-801, hsa-miR-192, hsa-miR-21 is up-regulated and the expression of any one or more of the nucleic acid molecules encoding hsa-miR-122, hsa-miR-26a, hsa-miR-27a, hsa-miR-223 is down-regulated in the one or more target plasma compared to the one or more controls and hsa-miR-1228 is un-changed in the one or more target plasma compared to the one or more control plasma.

The terms "any one or more of the plurality of nucleic acid molecules" or "any one or more of the plurality of nucleic acid molecules" as used herein, may relate to any subgroup of the plurality of nucleic acid molecules, e.g., any one, any two, any three, any four, any five, any six, any seven, any eight, any nine, any ten, and so forth nucleic acid molecules, each encoding at least one microRNA.

In most preferred embodiments, the plurality of nucleic acid molecules comprises a panel of eight nucleic acid molecules encoding hsa-miR-122 (SEQ ID NO:1), hsa-miR-192 (SEQ ID NO:2), hsa- miR-21 (SEQ ID NO:3), hsa- miR-223 (SEQ ID NO:4), hsa-miR-26a (SEQ ID NO:5), hsa-miR-27a (SEQ ID NO:6), hsa-miR-801 (SEQ ID NO:7) and hsa-miR-1228(SEQ ID NO:8). The panelof eight nucleic acid molecules is comprised by a logistic regression model: logit(p=HCC)=-1.424-0.292* hsa-miR-122+0.4511* hsa-miR-192+0.6112*hsa-miR-21-0.1796*hsa-miR-223-0.2487*hsa-miR-26a-0.3542*hsa-miR-27a+0.209* hsa-miR-801, wherein the expression levels of hsa-miR-122, hsa-miR-192, hsa-miR-21, hsa-miR-223, hsa-miR-26a, hsa-miR-27a and hsa-miR-801 are detected while using hsa-miR-1228 as endogenous control.

The nucleic acid sequences of the above-referenced microRNAs are listed in Table 1.

The expression of the logit(p=HCC) value from the model is up-regulated in the one or more target plasma compared to the one or more control plasma and the expression of hsa-miR-1228 is un-changed in the one or more target plasma compared to the one or more control plasma.

The term "nucleic acid panel", as used herein, refers to the usage of at least two nucleic acid expression levels as a whole. Preferably may use the relative changes or calculate results through a formulation as a whole.

In a second aspect, the present invention relates to a kit for diagnosing hepatocellular carcinoma, containing the marker for diagnosing hepatocellular carcinoma, the marker for diagnosing hepatocellular carcinoma consists of a plurality of nucleic acid molecules, each nucleic acid molecule encoding at least one microRNA sequence.

Particularly preferably, the plurality of nucleic acid molecules comprises one or more of the nucleic acid molecules encoding hsa-miR-122 (SEQ ID NO:1), hsa-miR-192 (SEQ ID NO:2), hsa-miR-21 (SEQ ID NO:3), hsa-miR-223 (SEQ ID NO:4), hsa-miR-26a (SEQ ID NO:5), hsa-miR-27a (SEQ ID NO:6), hsa-miR-801(SEQ ID NO:7) and an endogenous control hsa-miR-1228 (SEQ ID NO:8).

The nucleic acid sequences of the above-referenced microRNAs are listed in Table 1.

The expression of any one or more of the nucleic acid molecules encoding hsa-miR-801, hsa-miR-192, hsa-miR-21 is up-regulated and the expression of any one or more of the nucleic acid molecules encoding hsa-miR-122, hsa-miR-26a, hsa-miR-27a, hsa-miR-223 is down-regulated in the one or more target plasma compared to the one or more controls and hsa-miR-1228 is un-changed in the one or more target plasma compared to the one or more control plasma.

In most preferred embodiments, the plurality of nucleic acid molecules comprises a panel of eight nucleic acid molecules encoding hsa-miR-122 (SEQ ID NO:1), hsa-miR-192 (SEQ ID NO:2), hsa-miR-21 (SEQ ID NO:3), hsa-miR-223 (SEQ ID NO:4), hsa-miR-26a (SEQ ID NO:5), hsa-miR-27a (SEQ ID NO:6), hsa-miR-801 (SEQ ID NO:7) and hsa-miR-1228(SEQ ID NO:8). The kit also comprises a logistic regression model comprising the panel of eight nucleic acid molecules: logit(p=HCC)=-1.424-0.292* hsa-miR-122+0.4511*hsa-miR-192+0.6112*hsa-miR-21-0.1796*hsa-miR-223-0.2487*hsa-miR-26a-0.3542*hsa-miR-27a+0.209* hsa-miR-801, wherein the expression levels of hsa-miR-122, hsa-miR-192, hsa-miR-21, hsa-miR-223, hsa-miR-26a, hsa-miR-27a and hsa-miR-801 are detected while using hsa-miR-1228 as endogenous control.

The nucleic acid sequences of the above-referenced microRNAs are listed in Table 1.

The expression of the logit(p=HCC) value from the model is up-regulated in the one or more target plasma compared to the one or more control plasma and the expression of hsa-miR-1228 is un-changed in the one or more target plasma compared to the one or more control plasma.

In a third aspect, the present invention relates to a kit for diagnosing hepatocellular carcinoma, containing the marker for discriminating plasma of at least one hepatocellular carcinoma patient from that of at least one healthy individual, wherein the marker for diagnosing hepatocellular carcinoma consists of a plurality of nucleic acid molecules, each nucleic acid molecule encoding at least one microRNA sequence, the plurality of nucleic acid molecules comprises at least one nucleic acid molecule encoding the microRNA sequence differently expressed in one or more target plasma compared to one or more control plasma, and the control plasma are obtained from healthy individuals.

Particularly preferably, the plurality of nucleic acid molecules comprises one or more of the nucleic acid molecules encoding hsa-miR-122 (SEQ ID NO:1), hsa-miR-192 (SEQ ID NO:2), hsa-miR-21 (SEQ ID NO:3), hsa-miR-223 (SEQ ID NO:4), hsa-miR-26a (SEQ ID NO:5), hsa-miR-27a (SEQ ID NO:6), hsa-miR-801(SEQ ID NO:7) and an endogenous control hsa-miR-1228 (SEQ ID NO:8).

The nucleic acid sequences of the above-referenced microRNAs are listed in Table 1.

The expression of any one or more of the nucleic acid molecules encoding hsa-miR-801, hsa-miR-192, hsa-miR-21 is up-regulated and the expression of any one or more of the nucleic acid molecules encoding hsa-miR-122, hsa-miR-26a, hsa-miR-27a, hsa-miR-223 is down-regulated in the one or more target plasma compared to the one or more controls and hsa-miR-1228 is un-changed in the one or more target plasma compared to the one or more control plasma.

In most preferred embodiments, the plurality of nucleic acid molecules comprises a panel of eight nucleic acid molecules encoding hsa-miR-122 (SEQ ID NO:1), hsa-miR-192 (SEQ ID NO:2), hsa-miR-21 (SEQ ID NO:3), hsa-miR-223 (SEQ ID NO:4), hsa-miR-26a (SEQ ID NO:5), hsa-miR-27a (SEQ ID NO:6), hsa-miR-801 (SEQ ID NO:7) and hsa-miR-1228(SEQ ID NO:8). The kit also comprises a logistic regression model comprising the panel of eight nucleic acid molecules: logit(p=HCC)=-1.424-0.292* hsa-miR-122+0.4511* hsa-miR-192+0.6112*hsa-miR-21-0.1796*hsa-miR-223-0.2487*hsa-miR-26a-0.3542*hsa-miR-27a+0.209* hsa-miR-801, wherein the expression levels of hsa-miR-122, hsa-miR-192, hsa-miR-21, hsa-miR-223, hsa-miR-26a, hsa-miR-27a and hsa-miR-801 are detected while using hsa-miR-1228 as endogenous control.

The nucleic acid sequences of the above-referenced microRNAs are listed in Table 1.

The expression of the logit(p=HCC) value from the model is up-regulated in the one or more target plasma compared to the one or more control plasma and the expression of hsa-miR-1228 is un-changed in the one or more target plasma compared to the one or more control plasma.

In a forth aspect, the present invention relates to a kit for diagnosing hepatocellular carcinoma, containing the marker for discriminating plasma of at least one hepatocellular carcinoma patient from that of at least one healthy individual, wherein the marker for diagnosing hepatocellular carcinoma consists of a plurality of nucleic acid molecules, each nucleic acid molecule encoding at least one microRNA sequence, the plurality of nucleic acid molecules comprises at least one nucleic acid molecule encoding the microRNA sequence differently expressed in one or more target plasma compared to one or more control plasma, and the control plasma are obtained from, chronic hepatitis B patients.

Particularly preferably, the plurality of nucleic acid molecules comprises one or more of the nucleic acid molecules encoding hsa-miR-122 (SEQ ID NO:1), hsa-miR-192 (SEQ ID NO:2), hsa-miR-21 (SEQ ID NO:3), hsa-miR-223 (SEQ ID NO:4), hsa-miR-26a (SEQ ID NO:5), hsa-miR-27a (SEQ ID NO:6), hsa-miR-801(SEQ ID NO:7) and an endogenous control hsa-miR-1228 (SEQ ID NO:8).

The nucleic acid sequences of the above-referenced microRNAs are listed in Table 1.

The expression of any one or more of the nucleic acid molecules encoding hsa-miR-801, hsa-miR-192, hsa-miR-21 is up-regulated and the expression of any one or more of the nucleic acid molecules encoding hsa-miR-122, hsa-miR-26a, hsa-miR-27a, hsa-miR-223 is down-regulated in the one or more target plasma compared to the one or more controls and hsa-miR-1228 is un-changed in the one or more target plasma compared to the one or more control plasma.

In most preferred embodiments, the plurality of nucleic acid molecules comprises a panel of eight nucleic acid molecules encoding hsa-miR-122 (SEQ ID NO:1), hsa-miR-192 (SEQ ID NO:2), hsa-miR-21 (SEQ ID NO:3), hsa-miR-223 (SEQ ID NO:4), hsa-miR-26a (SEQ ID NO:5), hsa-miR-27a (SEQ ID NO:6), hsa-miR-801 (SEQ ID NO:7) and hsa-miR-1228(SEQ ID NO:8). The kit also comprises a logistic regression model comprising the panel of eight nucleic acid molecules: logit(p=HCC)=-1.424-0.292* hsa-miR-122+0.4511* hsa-miR-192+0.6112*hsa-miR-21-0.1796*hsa-miR-223-0.2487*hsa-miR-26a-0.3542*hsa-miR-27a+0.209* hsa-miR-801, wherein the expression levels of hsa-miR-122, hsa-miR-192, hsa-miR-21, hsa-miR-223, hsa-miR-26a, hsa-miR-27a and hsa-miR-801 are detected while using hsa-miR-1228 as endogenous control.

The nucleic acid sequences of the above-referenced microRNAs are listed in Table 1.

The expression of the logit(p=HCC) value from the model is up-regulated in the one or more target plasma compared to the one or more control plasma and the expression of hsa-miR-1228 is un-changed in the one or more target plasma compared to the one or more control plasma.

In a fisth aspect, the present invention relates to a kit for diagnosing hepatocellular carcinoma, containing the marker for discriminating plasma of at least one hepatocellular carcinoma patient from that of at least one healthy individual, wherein the marker for diagnosing hepatocellular carcinoma consists of a plurality of nucleic acid molecules, each nucleic acid molecule encoding at least one microRNA sequence, the plurality of nucleic acid molecules comprises at least one nucleic acid molecule encoding the microRNA sequence differently expressed in one or more target plasma compared to one or more control plasma, and the control plasma are obtained from, cirrhosis patients.

Particularly preferably, the plurality of nucleic acid molecules comprises one or more of the nucleic acid molecules encoding hsa-miR-122 (SEQ ID NO:1), hsa-miR-192 (SEQ ID NO:2), hsa-miR-21 (SEQ ID NO:3), hsa-miR-223 (SEQ ID NO:4), hsa-miR-26a (SEQ ID NO:5), hsa-miR-27a (SEQ ID NO:6), hsa-miR-801(SEQ ID NO:7) and an endogenous control hsa-miR-1228 (SEQ ID NO:8).

The nucleic acid sequences of the above-referenced microRNAs are listed in Table 1.

The expression of any one or more of the nucleic acid molecules encoding hsa-miR-801, hsa-miR-192, hsa-miR-21 is up-regulated and the expression of any one or more of the nucleic acid molecules encoding hsa-miR-122, hsa-miR-26a, hsa-miR-27a, hsa-miR-223 is down-regulated in the one or more target plasma compared to the one or more controls and hsa-miR-1228 is un-changed in the one or more target plasma compared to the one or more control plasma.

In most preferred embodiments, the plurality of nucleic acid molecules comprises a panel of eight nucleic acid molecules encoding hsa-miR-122 (SEQ ID NO:1), hsa-miR-192 (SEQ ID NO:2), hsa-miR-21 (SEQ ID NO:3), hsa-miR-223 (SEQ ID NO:4), hsa-miR-26a (SEQ ID NO:5), hsa-miR-27a (SEQ ID NO:6), hsa-miR-801 (SEQ ID NO:7) and hsa-miR-1228(SEQ ID NO:8). The kit also comprises a logistic regression model comprising the panel of eight nucleic acid molecules: logit(p=HCC)=-1.424-0.292* hsa-miR-122+0.4511* hsa-miR-192+0.6112*hsa-miR-21-0.1796*hsa-miR-223-0.2487*hsa-miR-26a-0.3542*hsa-miR-27a+0.209* hsa-miR-801, wherein the expression levels of hsa-miR-122, hsa-miR-192, hsa-miR-21, hsa-miR-223, hsa-miR-26a, hsa-miR-27a and hsa-miR-801 are detected while using hsa-miR-1228 as endogenous control.

The nucleic acid sequences of the above-referenced microRNAs are listed in Table 1.

The expression of the logit(p=HCC) value from the model is up-regulated in the one or more target plasma compared to the one or more control plasma and the expression of hsa-miR-1228 is un-changed in the one or more target plasma compared to the one or more control plasma.

In a sixth aspect, the present invention relates to a method for determining a marker for diagnosing hepatocellular carcinoma, comprising:
(a) determining in one or more target plasma the expression levels of a plurality of nucleic acid molecules, each nucleic acid molecule encoding at least one microRNA sequence;
(b) determining in one or more control plasma the expression levels of the plurality of nucleic acid molecules; and
(c) identifying from the plurality of nucleic acid molecules one or more nucleic acid molecules that are differentially expressed in the target plasma and the control plasma by comparing the respective expression levels obtained in steps (a) and (b) from the plurality of nucleic acid molecules, and identifying one or more target plasma exhibiting hepatocellular carcinoma by using the one or more nucleic acid molecules that are differentially expressed in the target plasma and the control plasma as a marker for diagnosing hepatocellular carcinoma.

The invention is further described by the figures and the following examples, which are solely for the purpose of illustrating specific embodiments of this invention, and are not to be construed as limiting the scope of the invention in any way.

### EXAMPLES

### Example 1: Plasma sample collection and preparation

The study in the invention was approved by the local ethics committee and informed consent was obtained from all patients. The study design in the invention for the discovery, training and validation phases of the microRNA biomarkers is shown in Figure 1. The principal method steps for identifying a patient in a blood sample using the proposed plasma microRNA panel exhibiting hepatocellular carcinoma are shown in Figure 2.

934 blood samples, who met the eligible criteria (Table 2), were prospectively collected from Shanghai Zhongshan Hospital and Public Health Center between August 2008 and June 2010. These samples were obtained from 167 healthy donors (healthy group), 169 chronic hepatitis B patients (CHB group), 141 post HBV infection liver cirrhosis patients (cirrhosis group) and 457 HBV-infection related HCC patients (HCC group). The samples were allocated into 3 phases of the study in chronological order (Figure 1). The clinical characteristics of the patients are summarized in Table 3 and 4.

**Table 2**

| Eligibility criteria for selection of the patients |
|---|
| General inclusion criteria |
| 1. Age ≤18 years and ≤90 years |
| 2. Not currently residing in an institution, such as a prison, nursing home, or shelter |
| 3. Not severely ill in the intensive care unit |
| 4. With the capability to give informed consent |
| 5. Encountered between August 2008 and June 2010 |

| Healthy donors (healthy group) |
|---|
| 1. Had the medical check-up in Zhongshan Hospital |
| 2. In general healthy condition without malignancy |

| Chronic hepatitis B patients (CHB group) |
|---|
| 1. Diagnosed with chronic hepatitis B (i.e., > 6 months in HBsAg+, HBeAg+ or HBeAg- but with detectable HBV DNA) |

| Post HBV infection liver cirrhosis patients (cirrhosis group) |
|---|
| 1. With HBV infection |
| 2. Diagnosed by two experienced pathologists |
| 3. If no tissue available, diagnosis must be supported by two image reports (ultrasound B, CT or MRI) |

| HBV-related HCC patients (HCC group) |
|---|
| 1. With HBV infection |
| 2. Diagnosed by two experienced pathologists |
| 3. If no tissue available, diagnosis must be supported by two image reports (ultrasound B, CT or MRI) and/or AFP |
| 4. No pre-operative chemotherapy, radiotherapy, transarterial chemoembolization or ablation |

**Table 3**

| Characteristics of study subjects on microarrays | | |
|---|---|---|
| Variable | | Discovery Phase (n=137) |
| Healthy count (%) | | |
| Age (Mean± SD) - yr | | 43 ± 14 |
| Sex - no. (%) | Male | 13 (39) |
| | Female | 20 (61) |
| ALT- no. (%) | ≤ 40 U/L | 25 (76) |
| | > 40 U/L | 3 (9) |
| | Missing | 5 (15) |
| AFP - no. (%) | ≤ 20 ng/ml | 33 (100) |
| | Missing | 0 |

| CHB Count (%) | | |
|---|---|---|
| Age (Mean± SD) - yr | | 42 ± 11 |
| Sex - no. (%) | Male | 18 (82) |
| | Female | 3 (14) |
| | Missing | 1 (5) |
| ALT- no. (%) | ≤ 40 U/L | 5 (23) |
| | > 40 U/L | 15 (68) |
| | Missing | 2 (9) |
| AFP - no. (%) | ≤ 20 ng/ml | 17 (77) |
| | > 20 ng/ml | 4 (18) |
| | Missing | 1 (5) |

| Cirrhosis count (%) | | |
|---|---|---|
| Age (Mean± SD) - yr | | 49 ± 11 |
| Sex - no. (%) | Male | 19 (76) |
| | Female | 6 (24) |
| ALT- no. (%) | ≤ 40 U/L | 10 (40) |
| | > 40 U/L | 11 (44) |
| | Missing | 4 (16) |
| AFP - no. (%) | ≤ 20 ng/ml | 21 (84) |
| | > 20 ng/ml | 4 (16) |
| | Missing | 0 |

| HCC count (%) | | |
|---|---|---|
| Age (Mean± SD) - yr | | 48 ± 10 |
| Sex - no. (%) | Male | 49 (86) |
| | Female | 8 (14) |
| ALT- no. (%) | ≤ 40 U/L | 22 (39) |
| | > 40 U/L | 27 (47) |
| | Missing | 8 (14) |
| AFP - no. (%) | ≤ 20 ng/ml | 18 (32) |
| | > 20 ng/ml | 39 (68) |
| | Missing | 0 |
| Tumor size - no. (%) | ≤ 3cm | 15 (26) |
| | > 3cm | 41 (72) |
| | Missing | 1 (2) |
| Multiple tumors - no. (%) | Yes | 12 (21) |
| | No | 44 (77) |
| | Missing | 1 (2) |
| BCLC stage - no. (%) | 0 | 5 (9) |
| | A | 8 (14) |
| | B | 35 (61) |
| | C | 8 (14) |
| | D | 0 |
| | Missing | 1 (2) |
| Edmonson grade - no. (%) | I, I-II or II | 30 (53) |
| | II-III or III | 23 (40) |
| | Missing | 4 (7) |
| Vascular invasion- no. (%) | Yes | 30 (53) |
| | No | 23 (40) |
| | Missing | 4 (7) |

**Table 4**

| Characteristics of study subjects in the straining and validation datasets | | | | |
|---|---|---|---|---|
| Variable | | Training ( n=407) | Validation (n=390) | P value |
| Healthy count (%) | | | | |
| Age (Mean± SD) | | 44 ± 11 | 45 ± 12 | 0.71 |
| Sex | Male | 35 (49) | 43 (65) | 0.11 |
| | Female | 33 (51) | 23 (35) | |
| ALT | ≤ 40 U/L | 56 (82) | 53 (80) | 0.76 |
| | > 40 U/L | 12 (18) | 13 (20) | |
| AFP | ≤ 20 ng/ml | 68 (100) | 66 (100) | |

| CHB count (%) | | | | |
|---|---|---|---|---|
| Age (Mean± SD) | | 39 ± 13 | 39 ± 14 | 0.93 |
| Sex | Male | 48 (64) | 35 (49) | 0.06 |
| | Female | 27 (36) | 37 (51) | |
| ALT | ≤ 40 U/L | 40 (53) | 42 (58) | 0.54 |
| | > 40 U/L | 35 (47) | 30 (42) | |
| AFP | ≤ 20 ng/ml | 60 (80) | 41 (57) | 0.003 |
| | > 20 ng/ml | 15 (20) | 31 (43) | |

| Cirrhosis count (%) | | | | |
|---|---|---|---|---|
| Age (Mean± SD) | | 53 ± 13 | 50 ± 10 | 0.13 |
| Sex | Male | 43 (72) | 40 (71) | 0.98 |
| | Female | 17 (27) | 16 (29) | |
| ALT | ≤ 40 U/L | 39 (65) | 22 (39) | 0.01 |
| | > 40 U/L | 21 (35) | 34 (61) | |
| AFP | ≤ 20 ng/ml | 55 (92) | 48 (56) | 0.31 |
| | > 20 ng/ml | 5 (8) | 8 (14) | |

| HCC count (%) | | | | |
|---|---|---|---|---|
| Age (Mean± SD) | | 53 ± 12 | 53 ± 12 | 0.95 |
| Sex | Male | 168 (82) | 166(85) | 0.53 |
| | Female | 36 (18) | 30 (15) | |
| ALT | ≤ 40 U/L | 135 (66) | 148 (76) | 0.04 |
| | > 40 U/L | 69 (34) | 48 (24) | |
| AFP | ≤ 20 ng/ml | 82 (40) | 79 (40) | 0.98 |
| | > 20 ng/ml | 122 (60) | 117 (60) | |
| Tumor size | ≤ 3cm | 71 (33) | 93 (45) | 0.02 |
| | > 3cm | 133 (66) | 106 (53) | |
| Multiple tumors | Yes | 32 (16) | 44 (22) | 0.25 |
| | No | 168 (82) | 151 (76) | |
| | Missing | 4 (2) | 4 (2) | |
| BCLC stage | 0 | 26 (13) | 36 (18) | 0.25 |
| | A | 132 (65) | 111 (57) | |
| | B | 31 (15) | 38 (19) | |
| | c | 14 (7) | 11 (6) | |
| | D | 1 (0.5) | 0 ( 0) | |
| Edmonson grade | I, or II | 138 ( 68) | 135 (69) | 0.78 |
| | III | 51 ( 25) | 50 (26) | |
| | Missing | 15 ( 7) | 11 ( 6) | |
| Vascular invasion | Yes | 67 (33) | 68 (35) | 0.50 |
| | No | 122 (60) | 122 (61) | |
| | Missing | 15 ( 7) | 9 ( 5) | |

Peripheral blood (4 ml) was drawn into EDTA tubes. Within 30 minutes, the tubes were subjected to centrifuge at 820g for 10 min. Then, 1-ml aliquots of the plasma was transferred to 1.5-ml tubes and centrifuged at 16,000g for 10 min to pellet any remaining cellular debris. Subsequently, the supernatant was transferred to fresh tubes and stored them at -80 °C.

Total RNA was extracted by using mirVana PARIS miRNA Isolation kit (AM1556, Applied Biosystems, Foster City, CA, USA) according to the instructions from the manufacturer (Ambion, Austin, TX). The concentration was quantified by NanoDrop 1000 Spectrophotometer (NanoDrop Technologies, Waltham, MA). A sample was discarded for further analyses if OD 260/280 ratio was less than 1.8.

### Example 2: microRNA microarray analysis

A qualitative analysis of the microRNA (differentially) expressed in a particular plasma sample may optionally be performed using the Agilent microRNA microarray platform (Agilent Technologies, Santa Clara, CA, USA). The microarray contains probes for 723 human microRNAs from the Sanger database v.10.1. Total RNA (100 ng) derived from each of 137 plasma samples were used as inputs for labeling via single-color Cy3 incorporation. Microarray slides were scanned by XDR Scan (PMT100, PMT5). The labeling and hybridization were performed according to the protocols in the Agilent microRNA microarray system. The microarray image information was converted into spot intensity values using Feature Extraction Software Rev. 9.5.3 (Agilent Technologies, Santa Clara, CA). The signals after background subtraction were normalized by a stable endogenous control hsa-miR-1228. After that, a log transform with base 2 was performed. A sample that showed intra-array coefficients of variation (CV) across replicated spots on an array above 15% or detectable signal less than 5% was considered to be unreliable and excluded from further analysis.

Subjects' demographic characteristics were reported with descriptive statistics. Chi-square or Student T test were used for the comparison between training and validation datasets (Table 5). The Kruskal-Wallis test was used for the overall comparison among HCC, healthy, CHB and cirrhosis groups. The Mann-Whitney unpaired test was used for between group comparisons. The p-values from those tests were all corrected for multiple comparisons with Benjamini-Hochberg method. All the p values were two sided.

Candidate microRNAs with detectable signals on microarrays were selected for the validation using the following criteria: 1) Corrected p value in the Kruskal-Wallis test amongst HCC, healthy, chronic hepatitis B (CHB) and cirrhosis groups was < 0.001. 2) Corrected p value in the Mann-Whitney unpaired test between HCC versus healthy groups was < 0.05. 3) Corrected p value in the Mann-Whitney unpaired test between HCC versus CHB groups was < 0.00000001 and 4) Corrected p value in the Mann-Whitney unpaired test between HCC versus cirrhosis was < 0.0001.

### Example 3: The microarray data on 137 samples

The expression profiles on the microarray analysis of 15 candidate microRNAs for further validation are shown in Table 5. The microRNA met the selection criteria is shown in bold.

**Table 5**

| Expression profiles of 15 candidate microRNAs on microarrays | | | | | | | | |
|---|---|---|---|---|---|---|---|---|
| | HCC versus control# | | HCC versus healthy | | HCC versus CHB | | HCC versus cirrhosis | |
| | p value | Fold change | p value | Fold change | p value | Fold change | p value | Fold change |
| hsa- miR-122 | 1.6E-10 | 0.3 | 1.7E-03 | 3.3 | 9.0E-09 | 0.1 | 8.8E-02 | 1.9 |
| hsa- miR- 801 | 2.8E-04 | 4.7 | 1.7E-03 | 2.4 | 4.0E-02 | 9.2 | 2.5E-04 | 9.1 |
| hsa- miR-194 | 2.7E-08 | 0.2 | 2.7E-03 | 6.7 | 6.5E-07 | 0.1 | 9.8E-03 | 6.6 |
| hsa-miR-223 | 4.1E-09 | 0.2 | 7.1E-03 | 0.5 | 5.8E-09 | 0.2 | 3.9E-06 | 0.2 |
| hsa-miR-21 | 7.6E-05 | 0.9 | 1.2E-02 | 2.0 | 2.8E-04 | 0.5 | 5.5E-01 | 1.2 |
| hsa-miR-23b | 9.4E-08 | 0.2 | 4.7E-02 | 3.9 | 9.9E-08 | 0.1 | 7.2E-01 | 0.8 |
| hsa-miR-192 | 2.7E-07 | 0.5 | 5.0E-02 | 2.9 | 9.7E-07 | 0.2 | 1.9E-02 | 4.6 |
| hsa-miR-101 | 2.7E-08 | 0.3 | 8.7E-01 | 0.9 | 5.8E-09 | 0.1 | 3.0E-02 | 1.4 |
| hsa-miR-122* | 1.9E-08 | 0.02 | 1.7E-01 | 2.6 | 5.8E-09 | 0.007 | 9.4E-01 | 0.7 |
| hsa-miR-19a | 3.5E-08 | 0.4 | 8.2E-01 | 0.8 | 5.8E-09 | 0.2 | 2.7E-02 | 2.6 |
| hsa-miR-19b | 8.4E-09 | 0.4 | 8.9E-01 | 0.9 | 5.8E-09 | 0.2 | 6.8E-03 | 2.0 |
| hsa-miR-29c | 1.3E-07 | 0.4 | 8.9E-01 | 0.8 | 5.8E-09 | 0.2 | 5.2E-01 | 1.0 |
| hsa- miR-27a | 4.0E-11 | 0.1 | 1.7E-01 | 0.5 | 6.6E-09 | 0.1 | 4.9E-09 | 0.1 |
| hsa-miR-181d | 1.4E-10 | 0.1 | 8.9E-01 | 0.8 | 3.9E-05 | 0.2 | 2.4E-09 | 0.1 |
| hsa- miR-26a | 8.9E-11 | 0.2 | 3.6E-01 | 0.5 | 8.7E-09 | 0.1 | 2.4E-09 | 0.1 |

### Example 4: Evaluation of 15 candidate microRNAs on 102 plasma samples

The evaluation of 15 candidate microRNAs discovered on microarrays was performed using an independent cohort of 102 plasma samples and different technology platform. An established quantitative RT-PCR employing a TaqMan MicroRNA assay kit (4427975, Applied Biosystems, Foster City, CA, USA) was used for the evaluation according to the manufacturer's instructions. All assays were carried out in triplicate. The expression level of hsa-miR-1228 was used as an endogenous control. A microRNA that showed CT values above 35 cycles in more than 20% of the 102 samples were excluded from further statistical analyses.

The Kruskal-Wallis test was used for the overall comparison among HCC, healthy, CHB and cirrhosis groups. The Mann-Whitney unpaired test was used for between group comparisons. The p-values from those tests were all corrected for multiple comparisons with Benjamini-Hochberg method. All the p values were two sided.

The expression profiles of 15 candidate microRNAs on quantitative RT-PCR are shown in Table 5. Of the 15 candidates, 12 microRNAs passed the quality control process. Seven of them (hsa-miR-122, hsa-miR-192, hsa-miR-21, hsa-miR-223, hsa-miR-26a, hsa-miR-27a and hsa-miR-801) had significant differential expression level between the HCC and control groups. The expression profiles on quantitative RT-PCR of 15 candidate microRNAs are shown in Table 6. Particularly preferred microRNAs (SEQ ID NO: 1 to SEQ ID NO: 7) are shown in bold.

**Table 6**

| Expression profiles of 15 candidate miRNAs on Quantitative RT-PCR in 102 samples | | | | | | | | |
|---|---|---|---|---|---|---|---|---|
| | HCC versus control# | | HCC versus healthy | | HCC versus CHB | | HCC versus cirrhosis | |
| | p value | Fold change | p value | Fold change | p value | Fold change | p value | Fold change |
| hsa-miR-122 | 1.5E-07 | 0.9 | 9.9E-04 | 0.9 | 2.2E-03 | 0.1 | 4.4E-01 | 0.7 |
| hsa-miR-801 | 1.1E-06 | 4.9 | 1.8E-05 | 4.9 | 1.3E-01 | 1.9 | 1.0E-04 | 5.9 |
| hsa- miR-194 | ND | ND | ND | ND | ND | ND | ND | ND |
| hsa-miR-223 | 1.4E-03 | 0.3 | 8.4E-01 | 0.3 | 4.5E-02 | 0.3 | 2.7E-03 | 0.1 |
| hsa-miR-21 | 1.1E-06 | 1.7 | 4.6E-04 | 1.7 | 1.8E-01 | 2.0 | 1.1E-01 | 0.3 |
| hsa- miR-23b | ND | ND | ND | ND | ND | ND | ND | ND |
| hsa-miR-192 | 1.1E-06 | 1.1 | 2.1E-04 | 1.1 | 1.3E-01 | 0.4 | 4.4E-01 | 0.5 |
| hsa-miR-101 | 7.6E-01 | 1.2 | 5.2E-01 | 1.2 | 9.6E-01 | 0.9 | 4.5E-01 | 1.9 |
| hsa-miR-122* | 8.7E-02 | 1.3 | 2.5E-01 | 1.3 | 7.0E-01 | 2.0 | 3.5E-01 | 0.4 |
| hsa-miR-19a | 7.6E-01 | 1.6 | 5.2E-01 | 1.6 | 3.9E-01 | 1.8 | 4.4E-01 | 2.1 |
| hsa-miR-19b | 7.6E-01 | 1.6 | 6.0E-01 | 1.6 | 5.1E-01 | 1.6 | 4.4E-01 | 2.4 |
| hsa-miR-29c | ND | ND | ND | ND | ND | ND | ND | ND |
| hsa-miR-27a | 3.8E-05 | 0.2 | 8.4E-01 | 0.2 | 1.4E-02 | 0.2 | 1.0E-04 | 0.1 |
| hsa- miR-181d | 2.3E-01 | 1.3 | 2.5E-01 | 1.3 | 9.0E-01 | 1.3 | 4.4E-01 | 0.6 |
| hsa-miR-26a | 2.6E-05 | 0.2 | 4.6E-01 | 0.2 | 3.2E-03 | 0.1 | 1.0E-04 | 0.1 |

| | | | | | | | | |
|---|---|---|---|---|---|---|---|---|
| #control group including healthy individuals, CHB patients and cirrhosis patients. The differentially expressed miRNA in HCC versus control is shown in bold. ND: not determined, the microRNA did not pass the quality control process. | | | | | | | | |

### Example 5: Establishing a predictive microRNA panel on training dataset containing 407 samples

The expression profile of the 7 differential expressed microRNAs was further evaluated on additional 305 plasma samples using the quantitative RT-PCR assays. A sample that showed CT values above 35 cycles in more than 20% of the 7 miRNAs were excluded from further analyses.

The combined 407 plasma samples were used as the training dataset for the construction of a microRNA panel in the diagnosis of HCC. Similarly, the Kruskal-Wallis test was used for the overall comparison among HCC, healthy, CHB and cirrhosis groups. The Mann-Whitney unpaired test was used for between group comparisons. The p-values from those tests were all corrected for multiple comparisons with Benjamini-Hochberg method. All the p values were two sided.

Stepwise logistic regression model was used to select prognostic microRNA markers based on the training dataset. The predicted probability of being diagnosed with HCC was used as a surrogate marker to construct receiver operating characteristic (ROC) curve. Area under the ROC curve (AUC) was used as an accuracy index for evaluating the diagnostic performance of serum AFP and the selected microRNA panel. MedCalc (10.4.7.0) software was used to perform the ROC and regression analyses.

The expression profiles and diagnostic performance of 7 candidate microRNAs in training dataset are shown in Table 6. The AUC for the microRNA panel was significantly larger than that of AFP (0.86 vs. 0.76, p<0.001, Figure 3A).

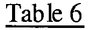

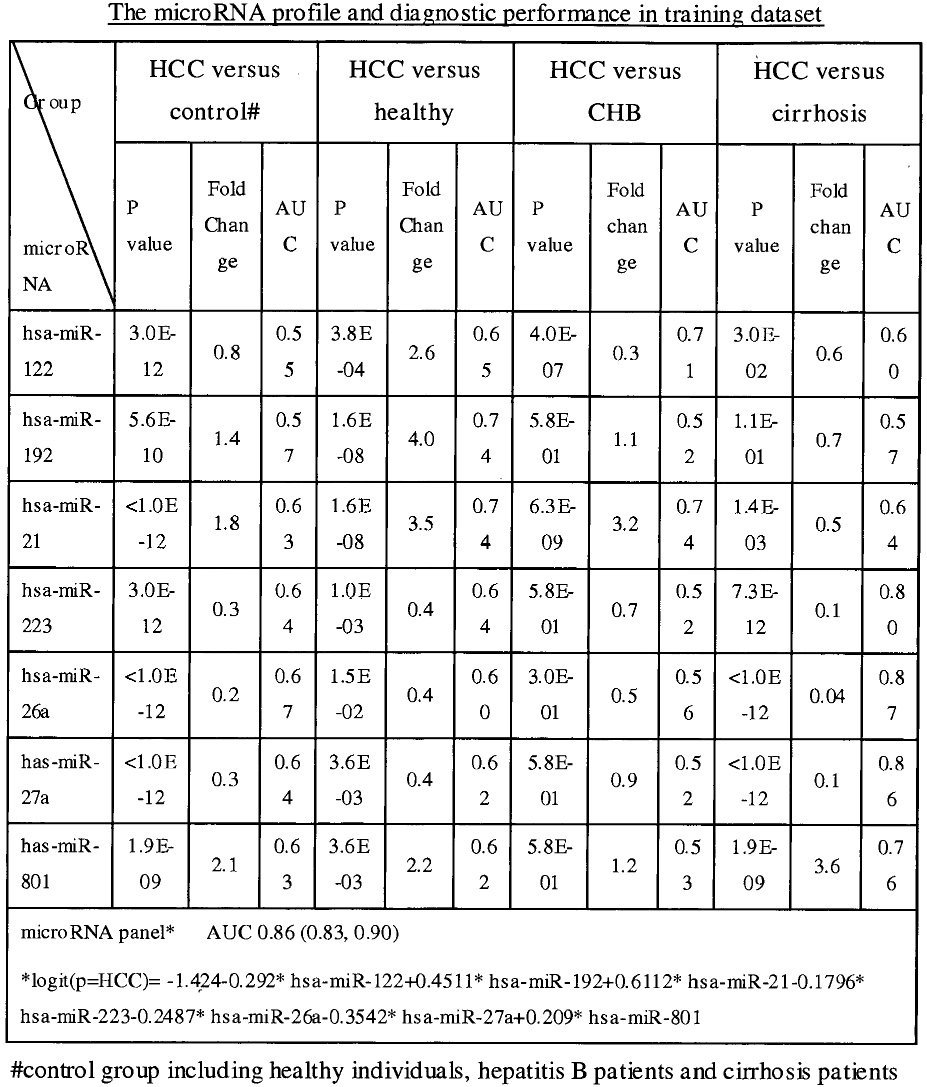

### Example 6: Validation of the microRNA panel on 390 validation dataset

The parameters estimated from the training dataset were used to predict the probability of being diagnosed with HCC on the independent validation dataset (390 plasma samples). Similarly, the experiments were performed using the quantitative RT-PCR assays. The predicted probability was used to construct the receiver operating characteristic curve.

The comparison of AUCs between the microRNA panel and AFP in the validation dataset indicated that the diagnostic accuracy of the microRNA panel was significantly better than that of AFP (AUC: 0.89 vs. 0.68, p<0.001, Figure 3B).

The comparison of the microRNA panel and AFP in discriminating HCC from healthy, CHB and cirrhosis groups was also performed (Figure 4). The analysis demonstrated that both microRNA panel and AFP can discriminate HCC from non-HCC subjects. However, the microRNA panel had significantly larger AUC than AFP (HCC vs. healthy: 0.95 vs. 0.64, p<0.001, HCC vs. CHB: 0.85 vs. 0.62, p<0.001 and HCC vs. cirrhosis: 0.89 vs. 0.78, p=0.002).

### Example 7: Diagnostic performance of the microRNA panel and AFP in the different BCLC stages

The diagnostic performance of the microRNA panel and AFP in the different BCLC stages was further evaluated (Figure 5). In term of diagnosing early and intermediate stages of HCC (BCLC stage 0, A and B), the microRNA panel had significantly better performance than AFP (for stage 0, AUC 0.89 vs. 0.62, p<0.001; for stage A, AUC 0.88 vs. 0.71, p<0.001; for BCLC stage B, AUC 0.91 vs. 0.63, p<0.001; Figure 5A, 5B and 5 C). There is no significant difference between the microRNA panel and AFP when the comparison of diagnostic performance was focused on BCLC stage C (AUC 0.88 vs. 0.77, p=0.24, figure 2f) patients.

### Example 8: Diagnostic performance of the microRNA panel in the low AFP (≤20ng/ml) and elevated AFP (>20ng/ml) groups

The diagnostic accuracy of the microRNA panel and AFP was then evaluated according to AFP level. In the low AFP (≤20ng/ml) group, the AUC of miRNA panel was significantly larger than that of AFP (0.87 vs. 0.63, p<0.001, Figure 5A). In the elevated AFP (>20ng/ml) group, the same trend was sustained (0.90 vs. 0.69, p<0.001, Figure 5B).

### Example 9: microRNA profile after HCC resection

Additionally, blood samples from 54 HCC patients, who received liver surgical resections, were obtained both preoperatively and the 6th days post operatively. The longitudinal changes of the expression profile of the microRNA panel were monitored using quantitative RT-PCR. All assays were carried out in triplicate. The expression level of hsa-miR-1228 was used as an endogenous control.

It was observed that the expression level of hsa-miR-21, hsa-miR-192 and AFP were significantly decreased after HCC resection (median difference 0.77, 1.74 and 6.66, p<0.001, p=0.03 and p<0.001 respectively, Figure 6). The post operative expression level of hsa-miR-223, hsa-miR-26a and hsa-miR-27a were elevated as compared to preoperative expression level (median difference: -0.61, -0.81 and -0.55, p=0.02, 0.06 and 0.06 respectively). The expression level of hsa-miR-122 and hsa-miR-801 did not have significant change following surgery.

The results obtained demonstrate a global highly specific regulation of microRNA expression in plasma of hepatocellular carcinoma patients. Thus, the respective validated subsets of microRNA specified herein represent unique microRNA biomarkers for expression profiling of hepatocellular carcinoma that do not only allow the early identification of a cancerogenous state as such but also enables the discrimination of hepatocellular carcinoma from chronic hepatitis B and cirrhosis.

The identification and validation of the microRNA expression biomarkers of the present invention provide a unique molecular marker that allows screening, early detection, differential diagnosing hepatocellular cancer in blood sample.

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. Use of a marker or of a kit containing said marker, consisting of a plurality of nucleic acid molecules, each nucleic acid molecule encoding at least one microRNA sequence, wherein the plurality of nucleic acid molecules comprises a panel of eight nucleic acid molecules encoding hsa-miR-122, hsa-miR-192, hsa-miR-21, hsa-miR-223, hsa-miR-26a, hsa-miR-27a, hsa-miR-801 and hsa-miR-1228 for diagnosing hepatocellular carcinoma.

2. The use of a marker or of a kit containing said marker according to claim 1, wherein the expressions of nucleic acid molecules encoding hsa-miR-801, hsa-miR-192 and hsa-miR-21 are up-regulated in one or more target plasma compared to one or more control plasma and the expressions of nucleic acid molecules encoding hsa-miR-122, hsa-miR-26a, hsa-miR-27a and hsa-miR-223 are down-regulated in one or more target plasma compared to one or more control plasma.

3. The use of a marker or of a kit containing said marker according to any one of claims 1 or 2, wherein the expression of nucleic acid molecules encoding hsa-miR-1228 is un-changed in one or more target plasma compared to one or more control plasma.

4. The use of a marker or of a kit containing said marker according to any one of claim 1-3, wherein the one or more control plasma are obtained from healthy individuals, chronic hepatitis B patients, or cirrhosis patients.

5. The use of a marker or of a kit containing said marker according to any one of claim 1-4, wherein the hepatocellular carcinoma is early hepatocellular carcinoma.

6. The use of the kit according to any one of claim 1-5, wherein the kit contains also a logistic regression model expressed below: logit(p=HCC)=-1.424-0.292*hsa-miR-122+0.4511*hsa-miR-192+0.6112*hsa-miR-21-0.1796*hsa-miR-223-0.2487*hsa-miR-26a-0.3542*hsa-miR-27a+0.209*hsa-miR-801, wherein the expression levels of hsa-miR-122, hsa-miR-192, hsa-miR-21, hsa-miR-223, hsa-miR-26a, hsa-miR-27a and hsa-miR-801 are detected while using hsa-miR-1228 as endogenous control, and the expression of the logit(p=HCC) value from the model is up-regulated in the one or more target plasma compared to the one or more control plasma.

7. Use of a kit containing the marker for diagnosing hepatocellular carcinoma according to claim 1 for discriminating plasma of at least one hepatocellular carcinoma patient from that of at least one healthy individual, or from that of at least one chronic hepatitis B patient, or from that of at least one cirrhosis patient, wherein the one or more control plasma are obtained respectively from healthy individuals, from chronic hepatitis B patients or from cirrhosis patients.

8. The use of a kit according to claim 7, containing also a logistic regression model expressed below: logit(p=HCC)=-1.424-0.292*hsa-miR-122+0.4511*hsa-miR-192+0.6112*hsa-miR-21-0.1796*hsa-miR-223-0.2487*hsa-miR-26a-0.3542*hsa-miR-27a+0.209*hsa-miR-801, wherein the expression levels of hsa-miR-122, hsa-miR-192, hsa-miR-21, hsa-miR-223, hsa-miR-26a, hsa-miR-27a and hsa-miR-801 are detected while using hsa-miR-1228 as endogenous control, and the expression of the logit(p=HCC) value from the model is up-regulated in the one or more target plasma compared to the one or more control plasma.

9. The use of a kit according to claim 8, wherein the hepatocellular carcinoma is early hepatocellular carcinoma.

10. A kit consisting in the panel of eight nucleic acid molecules encoding hsa-miR-122, hsa-miR-192, hsa-miR-21, hsa-miR-223, hsa-miR-26a, hsa-miR-27a, hsa-miR-801 and hsa-miR-1228, for diagnosing hepatocellular carcinoma.

11. The kit according to claim 10, wherein the hepatocellular carcinoma is early hepatocellular carcinoma.

## Patentansprüche

1. Verwendung eines Markers oder eines Kits, das den Marker enthält, der aus mehreren Nukleinsäuremolekülen besteht, wobei jedes Nukleinsäuremolekül wenigstens eine microRNA-Sequenz codiert, wobei die mehreren Nukleinsäuremoleküle eine Sammlung von acht Nukleinsäuremolekülen umfassen, die hsa-miR-122, hsa-miR-192, hsa-miR-21, hsa-miR-223, hsa-miR-26a, hsa-miR-27a, hsa-miR-801 und hsa-miR-1228 codieren, zum Diagnostizieren eines Leberzellkarzinoms.

2. Verwendung eines Markers oder eines Kits, das den Marker enthält, nach Anspruch 1, wobei die Expressionen der Nukleinsäuremoleküle, die hsa-miR-801, hsa-miR-192 und hsa-miR-21 codieren, in einem oder mehreren Zielplasma/-plasmen verglichen mit einem oder mehreren Kontrollplasma/-plasmen hochreguliert werden und die Expressionen der Nukleinsäuremoleküle, die hsa-miR-122, hsa-miR-26a, hsa-miR-27a und hsa-miR-223 in einem oder mehreren Zielplasma/-plasmen verglichen mit einem oder mehreren Kontrollplasma/-plasmen runterreguliert werden.

3. Verwendung eines Markers oder eines Kits, das den Marker enthält, nach Anspruch 1 oder 2, wobei die Expression der Nukleinsäuremoleküle, die hsa-miR-1228 codieren, in einem oder mehreren Zielplasma/-plasmen verglichen mit einem oder mehreren Kontrollplasma/-plasmen unverändert bleibt.

4. Verwendung eines Markers oder eines Kits, das den Marker enthält, nach einem der Ansprüche 1 bis 3, wobei das eine oder die mehren Kontrollplasma/-plasmen von gesunden Individuen, Patienten mit chronischer Hepatitis B oder Zirrhosepatienten gewonnen wird/werden.

5. Verwendung eines Markers oder eines Kits, das den Marker enthält, nach einem der Ansprüche 1 bis 4, wobei das Leberzellkarzinom ein Leberzellkarzinom im Frühstadium ist.

6. Verwendung des Kits nach einem der Ansprüche 1 bis 5, wobei das Kit auch ein unten dargestelltes Logistikregressionsmodel enthält: logit(p = HCC) = -1.424 - 0.292 * hsa-miR-122 + 0.4511 * hsa-miR - 192 + 0.6112 * hsa - miR - 21 - 0.1796 * hsa - miR - 223 - 0.2487 * hsa-miR - 26a - 0.3542 * hsa - miR - 27a + 0.209 * hsa - miR - 801,
wobei die Expressionslevel von hsa-miR-122, hsa-miR-192, hsa-miR-21, hsa-miR-223, hsa-miR-26a, hsa-miR-27a und hsa-miR-801 detektiert werden, während hsa-miR-1228 als endogene Kontrolle verwendet wird, und die Expression des logit(p=HCC)-Wertes von dem Model in einem oder mehreren Zielplasma/- plasmen verglichen mit einem oder mehreren Kontrollplasma/-plasmen hochreguliert wird.

7. Verwendung eines Kits, das den Marker zur Diagnose eines Leberzellkarzinoms enthält, nach Anspruch 1 zum Unterscheiden des Plasmas wenigstens eines Leberzellkarzinompatienten von dem wenigstens eines gesunden Individuums oder von dem wenigstens eines Patienten mit chronischer Hepatitis B oder von dem wenigstens eines Zirrhosepatienten, wobei das eine oder die mehreren Kontrollplasma/-plasmen von gesunden Individuen, Patienten mit chronischer Hepatitis B bzw. Zirrhosepatienten stammt/stammen.

8. Verwendung eines Kits nach Anspruch 7, wobei das Kit auch ein unten dargestelltes Logistikregressionsmodel enthält: logit(p = HCC) = -1.424 - 0.292 * hsa - miR - 122 + 0.4511 * hsa - miR-192 + 0.6112 * hsa - miR - 21 - 0.1796 * hsa - miR - 223 - 0.2487 * hsa - miR - 26a - 0.3542 * hsa-miR - 27a + 0.209 * hsa - miR - 801,
wobei die Expressionslevel von hsa-miR-122, hsa-miR-192, hsa-miR-21, hsa-miR-223, hsa-miR-26a, hsa-miR-27a und hsa-miR-801 detektiert werden, während hsa-miR-1228 als endogene Kontrolle verwendet wird, und die Expression des logit(p=HCC)-Wertes von dem Model in einem oder mehreren Zielplasma/- plasmen verglichen mit einem oder mehreren Kontrollplasma/-plasmen hochreguliert wird.

9. Verwendung eines Kits nach Anspruch 8, wobei das Leberzellkarzinom ein Leberzellkarzinom im Frühstadium ist.

10. Kit, das aus einer Sammlung von acht Nukleinsäuremolekülen besteht, die hsa-miR-122, hsa-miR-192, hsa-miR-21, hsa-miR-223, hsa-miR-26a, hsa-miR-27a, hsa-miR-801 und hsa-miR-1228 codieren, zum Diagnostizieren eines Leberzellkarzinoms.

11. Kit nach Anspruch 10, wobei das Leberzellkarzinom ein Leberzellkarzinom im Frühstadium ist.

## Revendications

1. Utilisation d'un marqueur ou d'un kit contenant ledit marqueur, consistant en une pluralité de molécules d'acide nucléique, chaque molécule d'acide nucléique codant au moins une séquence de microARN, où la pluralité de molécules d'acide nucléique comprend un ensemble de huit molécules d'acide nucléique codant hsa-miR-122, hsa-miR-192, hsa-miR-21, hsa-miR-223, hsa-miR-26a, hsa-miR-27a, hsa-miR-801 et hsa-miR-1228 pour le diagnostic de l'hépatome.

2. Utilisation d'un marqueur ou d'un kit contenant ledit marqueur selon la revendication 1, où les expressions de molécules d'acide nucléique codant hsa-miR-801, hsa-miR-192 et hsa-miR-21 sont régulées positivement dans un ou plusieurs plasmas cibles par rapport à un ou plusieurs plasmas témoins et les expressions de molécules d'acide nucléique codant hsa-miR-122, hsa-miR-26a, hsa-miR-27a et hsa-miR-223 sont régulées négativement dans un ou plusieurs plasmas cibles par rapport à un ou plusieurs plasmas témoins.

3. Utilisation d'un marqueur ou d'un kit contenant ledit marqueur selon l'une quelconque des revendications 1 ou 2, où l'expression de molécules d'acide nucléique codant hsa-miR-1228 est inchangée dans un ou plusieurs plasmas cibles par rapport à un ou plusieurs plasmas témoins.

4. Utilisation d'un marqueur ou d'un kit contenant ledit marqueur selon l'une quelconque des revendications 1-3, où le un ou plusieurs plasmas témoins sont obtenus auprès d'individus en bonne santé, de patients souffrant d'hépatite B chronique ou de patients souffrant de cirrhose.

5. Utilisation d'un marqueur ou d'un kit contenant ledit marqueur selon l'une quelconque des revendications 1-4, où l'hépatome est l'hépatome précoce.

6. Utilisation du kit selon l'une quelconque des revendications 1-5, où le kit contient aussi un modèle de régression logistique exprimé ci-dessous : logit(p=HCC)=-1,424-0,292*hsa-miR-122+0,4511*hsa-miR-192+0,6112*hsa-miR-21-0,1796*hsa-miR-223-0,2487*hsa-miR-26a-0,3542*hsa-miR-27a+0,209*hsa-miR-801, où les niveaux d'expression de hsa-miR-122, hsa-miR-192, hsa-miR-21, hsa-miR-223, hsa-miR-26a, hsa-miR-27a et hsa-miR-801 sont détectés tout en utilisant hsa-miR-1228 comme témoin endogène, et l'expression de la valeur logit(p=HCC) à partir du modèle est régulée positivement dans le un ou plusieurs plasmas cibles par rapport au un ou plusieurs plasmas témoins.

7. Utilisation d'un kit contenant le marqueur pour diagnostiquer un hépatome selon la revendication 1 pour distinguer le plasma d'au moins un patient souffrant d'hépatome de celui d'au moins un individu en bonne santé, ou de celui d'au moins un patient souffrant d'hépatite B chronique, ou de celui d'au moins un patient souffrant de cirrhose, où le un ou plusieurs plasmas témoins sont obtenus respectivement auprès d'individus en bonne santé, auprès de patients souffrant d'hépatite B chronique ou auprès de patients souffrant de cirrhose.

8. Utilisation d'un kit selon la revendication 7, contenant aussi un modèle de régression logistique exprimé ci-dessous : logit(p=HCC)= -1,424-0,292*hsa-miR-122+0,4511*hsa-miR-192+0,6112*hsa-miR-21-0,1796*hsa-miR-223-0,2487*hsa-miR-26a-0,3542*hsa-miR-27a+0,209*hsa-miR-801, où les niveaux d'expression de hsa-miR-122, hsa-miR-192, hsa-miR-21, hsa-miR-223, hsa-miR-26a, hsa-miR-27a et hsa-miR-801 sont détectés tout en utilisant hsa-miR-1228 comme témoin endogène, et l'expression de la valeur logit(p=HCC) à partir du modèle est régulée positivement dans le un ou plusieurs plasmas cibles par rapport au un ou plusieurs plasmas témoins.

9. Utilisation d'un kit selon la revendication 8, où l'hépatome est l'hépatome précoce.

10. Kit consistant en l'ensemble de huit molécules d'acide nucléique codant hsa-miR-122, hsa-miR-192, hsa-miR-21, hsa-miR-223, hsa-miR-26a, hsa-miR-27a, hsa-miR-801 et hsa-miR-1228 pour diagnostiquer un hépatome.

11. Kit selon la revendication 10, où l'hépatome est l'hépatome précoce.


### SEQUENCE LISTING

<110> Liver Cancer Institute, Zhongshan Hospital, Fudan University, Shanghai, China
<120> MARKER CONSISTING OF PLASMA MICRORNA AND A NEW METHOD FOR DIAGNOSIS OF HEPATOCELLULAR CARCINOMA
<130> XXXXX EP1
<150 > CN XXXXXXXX
   <151> 2011XX-XX
<160> 8
<170> PatentIn version 1.1
<210> 1
   <211> 22
   <212> RNA
   <213> Homo sapiens
<220>
   <221> misc_feature
   <223> hsa-miR-122 microRNA
<400> 1
   uggaguguga caaugguguu ug 22
<210> 2
   <211> 22
   <212> RNA
   <213> Homo sapiens
<210> 2
   <211> 21
   <212> RNA
   <213> Homo sapiens
<220>
   <221> misc_feature
   <223> hsa-miR-192 microRNA
<400> 2
   cugaccuaug aauugacagc c 21
<210> 3
   <211> 22
   <212> RNA
   <213> Homo sapiens
<220>
   <221> misc_feature
   <223> hsa-miR-21 microRNA
<400> 3
   uagcuuauca gacugauguu ga 22
<210> 4
   <211> 22
   <212> RNA
   <213> Homo sapiens
<220>
   <221> misc_feature
   <223> hsa-miR-223 microRNA
<400> 4
   ugucaguuug ucaaauaccc ca 22
<210> 5
   <211> 22
   <212> RNA
   <213> Homo sapiens
<220>
   <221> misc_feature
   <223> hsa-miR-26a microRNA
<400> 5
   uucaaguaau ccaggauagg cu 22
<210> 6
   <211> 21
   <212> RNA
   <213> Homo sapiens
<220>
   <221> misc_feature
   <223> hsa-miR-27a microRNA
<400> 6
   uucacagugg cuaaguuccg c 21
<210> 7
   <211> 24
   <212> RNA
   <213> Homo sapiens
<220>
   <221> misc_feature
   <223> hsa-miR-801 microRNA
<400> 7
   gauugcucug cgugcggaau cgac 24
<210> 8
   <211> 20
   <212> RNA
   <213> Homo sapiens
<220>
   <221> misc_feature
   <223> hsa-miR-1228 microRNA
<400> 20
   ucacaccugc cucgcccccc 20